# EUROPEAN PATENT APPLICATION

(11) **EP 1 308 517 A1**
(43) Date of publication of application: **07.05.2003**
(21) Application number: 01402830.2
(22) Date of filing: 31.10.2001
(51) Int. Cl.: C12N 15/85, C12N 15/67, C07K 14/55, C07K 14/535, C12N 9/12, C12N 5/10, C12N 15/26, C12N 15/27, C12N 15/54, A61K 48/00, G01N 33/50

(54) **Vectors for expressing multiple transgenes**

(71) Applicant: Aventis Pharmacueticals Products Inc., Bridgewater, NJ 08807-0800 (US)
(72) Inventor: Diagana, Melissa, San Francisco, CA 94110 (US); Brockstedt, Dirk, Oakland, CA 94610 (US)
(74) Representative: Bouvet, Philippe

(57) **Abstract**

The invention relates to recombinant vectors, expression cassettes, and nucleic acids, the use of which enables the expression of multiple transgenes from a single vector. Preferably, the vectors are plasmids, adeno-associated virus, or adenovirus vectors that can be used to infect and create mammalian cells in order to express multiple transgenes. Typically, expression systems and methods for expressing multiple transgenes require the use of more than one vector or the use of an internal ribosome entry site (IRES). For a number of reasons, the new vectors, expression cassettes, nucleic acids, and methods of the invention provide advantages over these other expression systems.

## Description

### Field of the Invention and Introduction

The invention relates to recombinant vectors, expression cassettes, and nucleic acids, the use of which enables the expression of multiple transgenes from a single vector. Preferably, the vectors are plasmids, adeno-associated virus, or adenovirus vectors that can be used to infect and create mammalian cells in order to express multiple transgenes. Typically, expression systems and methods for expressing multiple transgenes require the use of more than one vector or the use of an internal ribosome entry site (IRES). For a number of reasons, the new vectors, expression cassettes, nucleic acids, and methods of the invention provide advantages over these other expression systems.

More specifically, in one aspect, the invention provides a recombinant expression cassette that comprises two transgenes oriented in opposite directions with respect to their reading frames. Comparative examples, where the transgenes are oriented in the same direction, do not possess the properties of the vectors of this invention, i.e., higher levels of expression, and/or relatively equal amounts of each of the expressed transgenes from the expression cassette, and/or use in mammalian systems. The vectors of the invention can be advantageously used where equal levels of two or more transgenes are desired. For example, two chain proteins require the expression of two separate sequences in a single cell to improve the yield of functional, properly folded protein. In addition, combinations of bioactive polypeptides or combinations of polypeptides that influence similar pathways can also be expressed in the same cell. The vectors, expression cassettes, nucleic acids, and methods of the invention are not limited to use in any particular vector or viral system, however.

### Discussion of Related Technology

Previous attempts to have cells express two or more separate protein or polypeptide chains have used multiple vectors, each encoding a single gene. They have also employed vectors with an internal ribosome entry site (IRES). There are significant drawbacks for those approaches, however. First, it is difficult if not impossible to ensure that a representative number of cells will be infected or transfected with the same, or roughly the same, number of each of the vectors when multiple vectors are used. As a result, the levels of expression of one protein chain compared to another can vary drastically simply because a variable number of vectors exist within or become incorporated into different cells.

Similarly, the use of an IRES produces radically disparate protein levels. As known in the art, the sequence downstream of the IRES is expressed at much lower levels than the sequence upstream. *See, for example,* Mizuguchi, *et al., Mol. Ther. 1:* 376-382 (2000). Clearly, the art is in need of more flexible and more efficient vectors and vector systems and methods for expressing more than one polypeptide chain within a single cell.

### Summary of the Invention

The invention comprises recombinant expression cassettes and vectors capable of expressing two or more separate transgenes or polypeptides from a single vector. In addition, the invention comprises methods of using the recombinant expression cassettes and vectors to produce cells, extracts, or cell-derived preparations that contain the transgenes or polypeptides encoded by the cassette or vector or contained in the vector itself. Similarly, cells, tissues, and organisms bearing the expression cassettes and vectors are an aspect of the invention. Methods for producing recombinant vectors are also included within the invention. Various non-limiting aspects and embodiments are described below.

In one aspect, the invention comprises a recombinant vector capable of expressing two separate transgenes or polypeptides, where the two transgenes or polypeptides can be expressed at relatively equal levels. The level of expression can be up to ten-fold different between a first and second polypeptide, or a first polypeptide compared to any other number of expressed polypeptides. More preferably, the difference in expression levels between a first and second transgene or polypeptide can be from about five-fold to about two-fold. Most preferably, the difference in expression levels between a first and second transgene or polypeptide can be from about 100% to about 75%, or from about 100% to about 50%, or from about 40% to about 10%. Cells, tissue, and organisms containing these vectors are another aspect of the invention.

An aspect of the invention also comprises an expression cassette for expressing two or more transgenes that can be incorporated into a variety of vectors for administration to a cell or animal. The expression cassette contains the sequences of two or more transgenes linked to appropriate and/or desirable regulatory regions, so that the transgenes can be transcribed into RNA and/or a gene product. The sequences of two of the transgenes are oriented in opposite directions with respect to their reading frames or their initiation and termination sites. Preferably, two transgenes are selected for use and placed in an end-to-end fashion in the expression cassette, so that the poly A or terminus site associated with one transgene is positioned near the poly A site or terminus of a second transgene. Preferably, the sequences between the poly A are not regulatory sequences or regulatory regions. One of skill in the art is familiar with the use and construction of expression cassettes in general. The orientation and selection of cassette sequences disclosed here advances the art and provides new designs for improved gene transfer and expression methods.

The expression cassettes of the invention can be used in a variety of vectors and in a variety of methods for treating disease, expressing proteins or polypeptides, and analyzing the functional activity of proteins or transcribed sequences, for example. One skilled in the art is familiar with numerous ways in which the expression cassettes of the invention can be incorporated into vectors, used to cause the expression of proteins or polypeptides in selected cells or animals, and used to add or modify a biological function or characteristic of the cell or animal the vector is administered to. For example, the expression cassettes and vectors can be used to express transgenes in particular cells to give rise to therapeutic, prophylactic, or ameliorative effects.

In another aspect, the invention comprises a recombinant vector capable of expressing two separate polypeptide chains, where the sequences encoding the polypeptide chains are oriented in opposite directions. In preferred embodiments, the vector is capable of being used for gene transfer into a mammalian cell, so that the mammalian cell expresses the two polypeptide chains at levels that can be detected. In another embodiment, the polypeptides can be detected as a result of their functional characteristic(s), bioactivity characteristic(s), or structural characteristic(s). For example, the polypeptides can be detected by antibody-based assays, such as ELISA, FACS, or RIA. The cells may express polypeptides or proteins that allow them to destroy or affect tumor cells or proliferating cells so that a change in the tumor cell or proliferating cell growth pattern or viability characteristic(s) is detectable. One skilled in the art is familiar with numerous methods to detect changes in cell growth or viability. Other examples of detecting characteristics of expressed proteins or polypeptides can be used in combination with the invention.

In another aspect, the invention comprises nucleic acids that comprise two or more transgenes or polypeptide-encoding nucleic acid sequences, such as DNA, genomic DNA, cDNA, cDNA-derived nucleic acid, or RNA where retroviruses and other applications are involved, where the sequences are operably linked to regulatory or control elements, and wherein the orientation of one transgene or polypeptide-encoding nucleic acid is in the opposite direction, with respect to their reading frames or start and stop sites, compared to at least one other transgene or polypeptide-encoding sequence. Typically, these nucleic acids will be capable of being used in a cassette form, so that the transgene or polypeptide encoding sequences and linked to regulatory or control elements are present on a single nucleic acid. In a preferred embodiment, a nucleic acid of the invention relates to a cassette comprising two polypeptide-encoding nucleic acids, where the two polypeptide-encoding sequences are in opposite orientation with respect to their reading frames, and where the two sequences, and their linked regulatory or control elements, are present on a single cassette. The regulatory or control elements or regions are those sequences that allow for the appropriate expression, such as transcription, processing, and/or translation into polypeptides or proteins encoded in a selected cell. As one skilled in the art knows, promoters, promoter/enhancers, enhancers, donor/acceptor splice sites from intron sequences, poly-A addition sites, and other regulatory or control elements can be used on a nucleic acid or vector. These regulatory or control elements can be selected for use in a particular cell or organism. For example, a promoter/enhancer sequence that results in high levels of expression in mammalian cells would be desirable for producing proteins or transgenes in a mammalian cell.

In another aspect, the invention comprises methods for producing a cell that expresses two or more transgenes or polypeptides from a single recombinant vector. The recombinant vector can be one of those generally described above. By a "single" vector, we refer to an individual molecule, such as an individual viral particle or viral nucleic acid, or a number of molecules each of the same vector.

One method of the invention comprises introducing a recombinant vector or nucleic acid into a cell. Various means and methods for introducing vectors into a cell are known in the art, and the method of introduction itself does not limit the invention here. However, preferred methods of introducing the vector include adding the vector to cultured cells, injecting the vector into certain areas, tissue, or cells of an animal, using microprojectile methods, using liposomes or other deliver molecules or devices, electroporation methods, or administering the vector to an animal in appropriate vehicles and compositions, for example.

In another aspect, the invention comprises methods for producing cells capable of expressing transgenes or polypeptides possessing bioactivity and/or therapeutic activity. The methods comprise introducing a vector or nucleic acid of the invention into a cell. Bioactive and/or therapeutic activities can, for example, be detected by analyzing the affect of the expressed transgenes or polypeptides or be deduced from the presence of detectable transgenes or polypeptides. The cells can be used within an animal to determine the response or effect of the expressed transgenes or polypeptides on the animal or on a particular system, biological pathway, tissue or cell of the animal. The cells can be produced by administering or injecting a vector or nucleic acid of the invention into an animal.

In another aspect, the vectors, nucleic acids, methods, and cells of the invention can be used as part of a treatment regimen for an animal, including humans, either alone or in combination with therapeutic compounds or other treatments, or with pharmaceutically acceptable vehicles.

In particular, the invention comprises adenoviral vectors that contain at least two polypeptide-encoding DNA sequences, wherein two of the polypeptide-encoding DNA sequences are arranged in opposite orientations with respect to their reading frames. The polypeptide-encoding DNA sequences can be prepared from separate cassettes, together with their respective promoter/enhancer and other regulatory regions, or they can be present on a single expression cassette. Preferably, the polypeptide-encoding DNA sequences are inserted into an adenoviral genome within a deletion in the E1, E4, E2, or E3 region. However, other insertions and other insertions and deletions are possible. For example, replication deficient adenoviral vectors of various types can be selected as well as replication competent adenoviral vectors, and conditionally replicative adenoviral vectors, where replication depends on the absence or level of functionally active, endogenous host cell proteins such as p53, Rb, or other proteins related to tumor suppression, cell growth or apoptotic pathways. Many types of adenoviral vectors have been described in the art.

In another particular embodiment, the invention comprises a nucleic acid comprising, consisting essentially of, or consisting of two or more bioactive polypeptide-encoding and promoter-linked DNA sequences, wherein two of the polypeptide-encoding sequences are arranged in opposite orientation with respect to their reading frames, and wherein the nucleic acid does not comprise an IRES sequence. The nucleic acid can be capable of or suitable for insertion into or incorporation into a vector, plasmid, cosmid, BAC, YAC, viral genome or vector, a recombinant adenoviral vector, or other nucleic acid that integrates or resides within a cell. Preferably, the bioactive polypeptide-encoding sequences are not plasmid marker genes, such as tetracycline or antibiotic resistance genes and the like, and are not reporter genes, such as CAT, β-gal, and alkaline phosphatase. Reporter transgenes can be useful in the determining the level and location of transgene expression, however. The use of HSV-TK as a transgene, for example, allows non-invasive imaging techniques to correlate TK expression levels and presence with the expression levels of other transgenes. In this way, TK transgenes can be selected as either or both of a biologically active transgene product in the transduced cell and a reporter for the expression of other transgenes present on the same or similarly introduced vector. Other reporter transgenes, such as alkaline phosphatase or truncated forms of alkaline phosphatase, can also be selected and used in the expression cassettes. The expression cassettes and vectors of the invention are particularly useful in this regard since TK can be inserted into the same vector as other transgenes. Furthermore, the levels of TK can be correlated with those of other transgenes. Other reporter genes can be utilized in equivalent ways.

In another embodiment, the invention comprises a method of introducing a viral vector, plasmid vector, or vector containing a nucleic acid of the invention into a cell. The method may utilize any available technology or device for introduction of the vector. The viral vector can be, for example, an adenoviral vector, an adeno-associated virus vector, lentiviral, or retroviral vector, for example. These methods can be used to treat various conditions, improve or produce immunity to a tumor antigen or cell, inhibit cell growth, enhance cell growth, produce a reporter gene at a site of introduction, or reduce metastasis, for example.

The preparation of cells expressing two or more bioactive or functional polypeptides or proteins has become important for a number of reasons and is an important aspect of this invention. For example, the cells can be used to prepare or secrete polypeptides with complex tertiary structures or polypeptides composed of two or more chains. One of the many examples possible is IL-12, which contains two separate chains. The expression of multiple polypeptides may also result in synergistic functional effects. Therefore, cells expressing multiple immune response-activating polypeptide-encoding sequences can be used instead of multiple cells expressing individual sequences. Furthermore, combinations of functional sequences, a cytokine and a cytotoxic protein or polypeptide functional sequence, or multiple cytotoxicity-encoding sequences, can be inserted into cells. And, in an important embodiment of the invention, the vectors, cassettes, nucleic acids, or methods of the invention can be employed for functional characterization and screening of mutated or novel sequences or the characterization of sequences lacking functional annotations, as for example sequences derived from or identified from an EST, or genomic database, or an expression profiling or other functional genomic screen.

### Description of the Figures

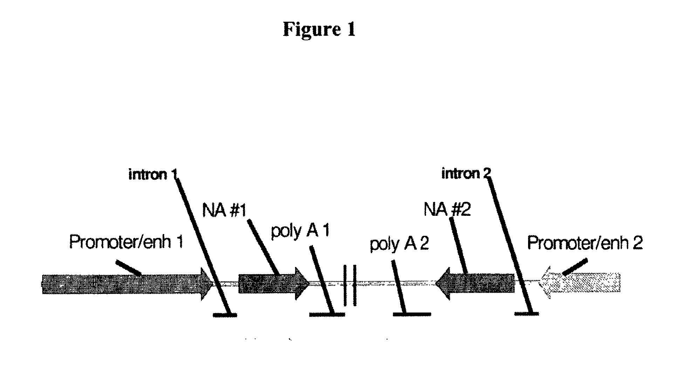

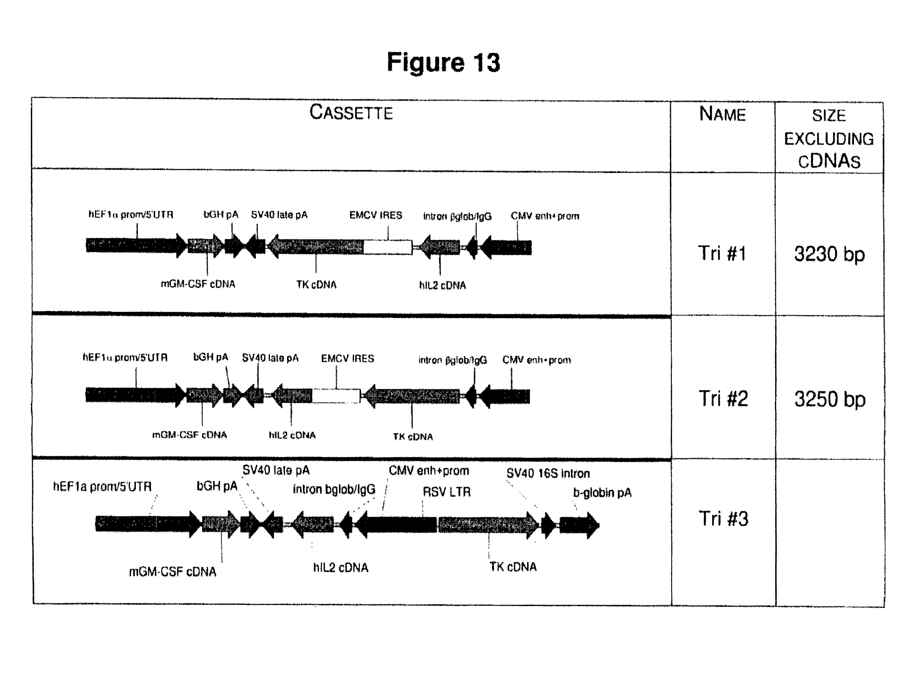

Figure 1 depicts a schematic representation of regulatory and polypeptide-encoding or transgene elements present in an exemplary embodiment of the invention, comprising two separate protein-encoding nucleic acids or transgenes. For example, the transgenes or protein-encoding sequences, NA #1 and NA #2 (nucleic acid #1 and #2), and their respective promoter and enhancer elements and intron elements are oriented in opposite directions. Promoter/enhancer, intron sites, and poly A sites can be the same or different. The NA #1 and #2, the separate gene or polypeptide-encoding sequences, need not be cDNA, cDNA-derived, or genomic sequences. Any form of nucleic acid, DNA, RNA, synthetic nucleic acid, or combinations, depending on the use selected or intended, may be used. They can also be the same gene or polypeptide-encoding sequence, or separate chains of a complex or two or more chain-containing protein. They can also be transgenes that produce functional transcripts, such as anti-sense nucleic acids or ribozymes. The double lines between the two poly A sites represents the point at which the orientation of elements related to each transgene become opposite with respect to the reading frames. As shown in Figure 13, below, additional regions for expressing additional transgenes can be inserted into these general configurations. Some of the additional transgenes can employ an IRES to drive expression from the third or other multiple transgene sequence.

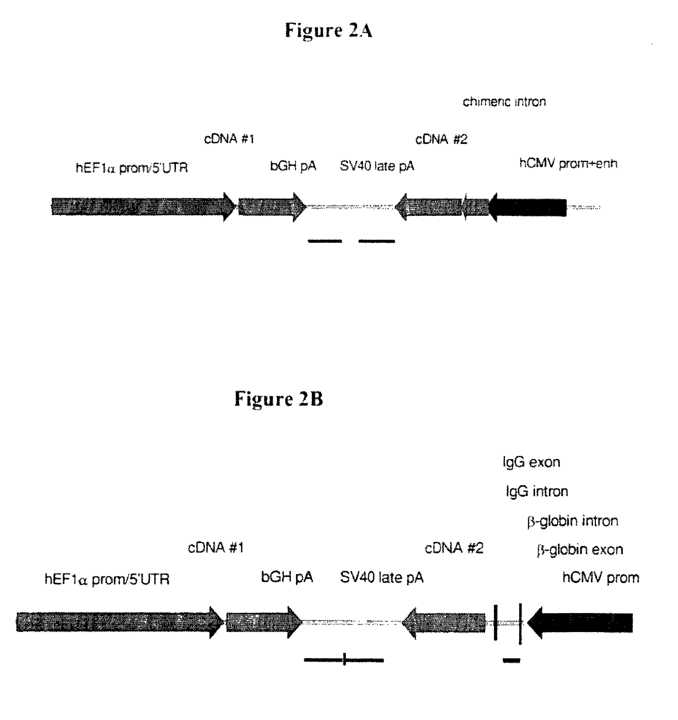

Figure 2A depicts a schematic representation of exemplary promoter/enhancer and poly A regulatory elements in a single, end-to-end cassette embodiment of the invention. In Figures 2A and 2B, the transgenes or bioactive polypeptide-encoding sequences are listed as "cDNA." However, the invention clearly encompasses other nucleic acids containing the same sequence information and control regions, for example, RNA or dsRNA when used in a viral vector that contains RNA. Again, additional transgenes expressing regions can be incorporated into this constuct to create three-gene or multiple gene cassettes.
Figure 2B depicts a schematic representation of an exemplary expression cassette design. The promoter/enhancer elements are selected for high expression. Here the hEF1α prom/5'UTR contains an intron splice donor and acceptor sequence. The regulatory elements can be selected from various plasmids, which can also be used to construct the recombinant vectors of the invention. Again, additional transgene expressing regions can be incorporated into this construct to create three-gene or multiple gene cassettes.

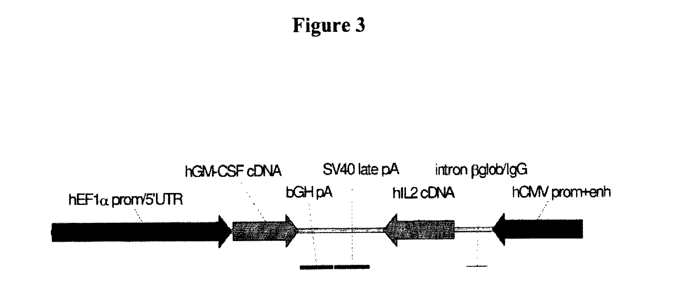

Figure 3 depicts the exemplary expression cassette design employed in Example 2. Polypeptide-encoding sequences for GM-CSF and IL2 are linked to promoter enhancer elements in opposite orientations. The human, murine, or other mammalian homologue of these genes can be selected, depending of the intended use of the cassette. Both polypeptide-encoding sequences are contained in a single cassette. This cassette can be inserted into various regions of an appropriate vector. For example, this expression cassette can be inserted into, or inserted into deletions of, the entire E1 region, or the E1a, E1b, E2, E4, or E3 region of an adenovirus, such as human Ad-2 or Ad-5, to produce a recombinant vector of the invention. It can also be sued to create conditionally replicative adenovirus vectors.

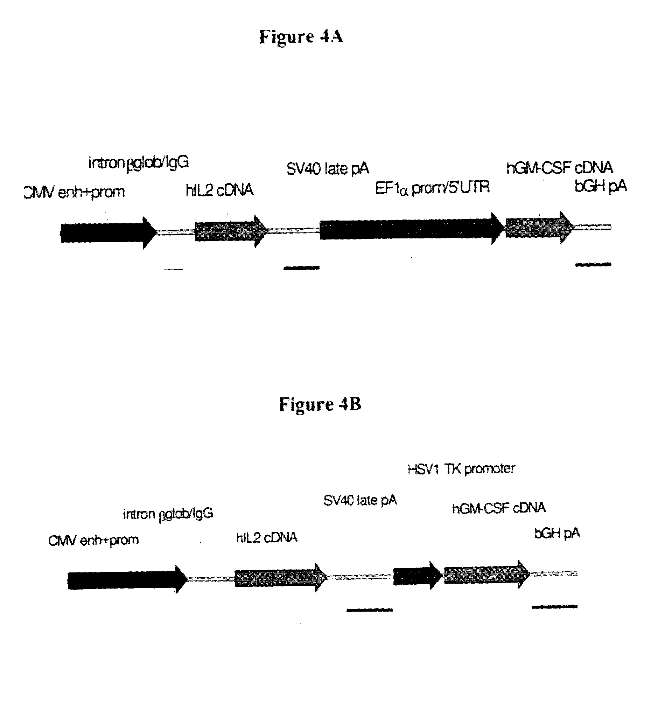

Figure 4A depicts a comparative example, wherein the polypeptide-encoding sequences are oriented in the same direction.
Figure 4B depicts another comparative example, wherein the polypeptide-encoding sequences are oriented in the same direction.

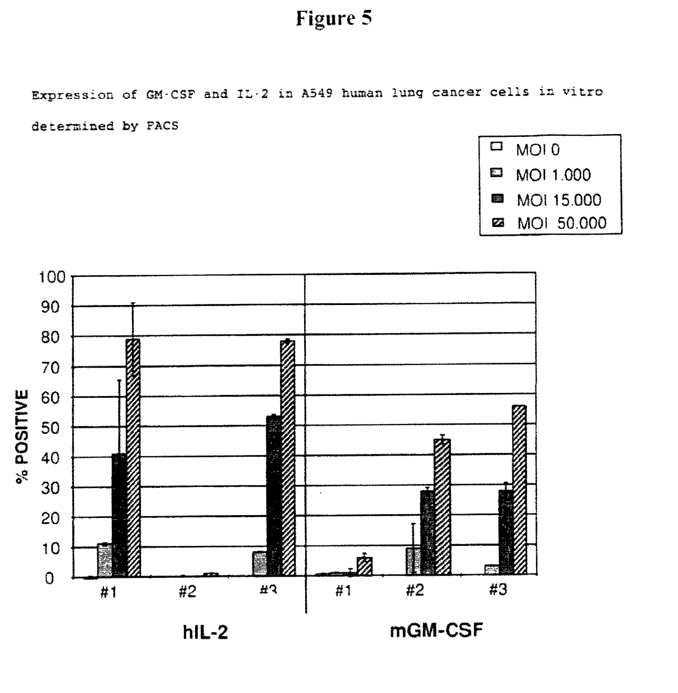

Figure 5 depicts the number of cells (% positive cells) expressing human IL-2 and murine GM-CSF (in A549 human lung carcinoma cells; ATCC CCL 185) at various viral particles per cell (MOI) as determined by fluorescence activated cell-sorting (FACS). A vector of the invention, containing both the IL-2 and GM-CSF coding regions, arranged in opposite orientation and without an IRES between each of the two protein-encoding sequences, was used.

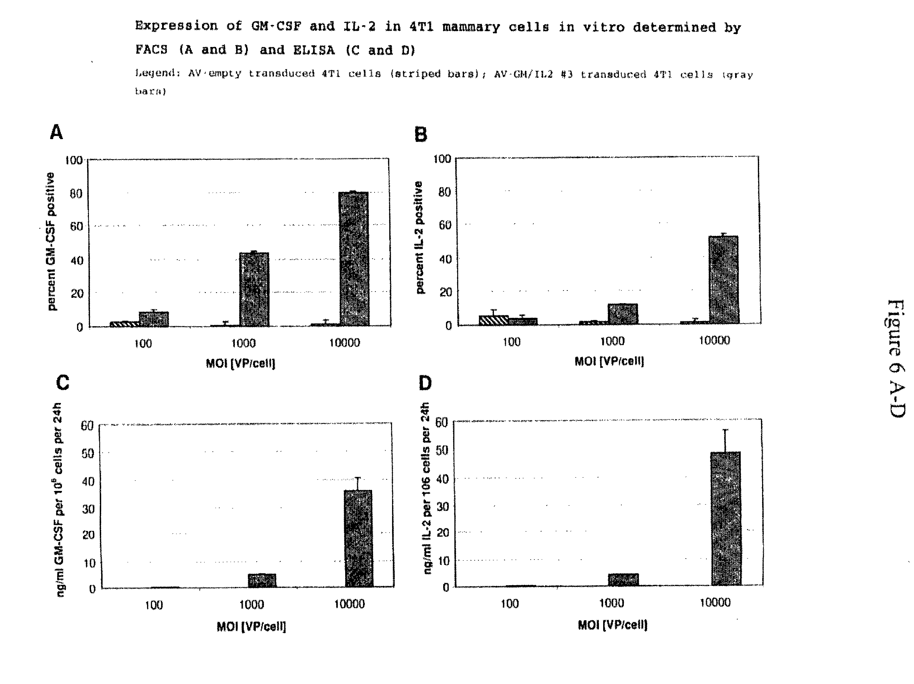

Figure 6 depicts the percentage of cells expressing GM-CSF (Figure 6A) and IL-2 (Figure 6B) at various MOI as determined by FACS, and depicts the level of protein secreted into the medium of those cells (Figures 6C and 6D) as determined by ELISA. At lower MOI, the difference in expression level is very low, on the order of 10% or less than 20%. At higher MOI, for example 10,000 virus particles per cell (VP/cell), the difference in expression level is approximately 33.3% or less than 40%.

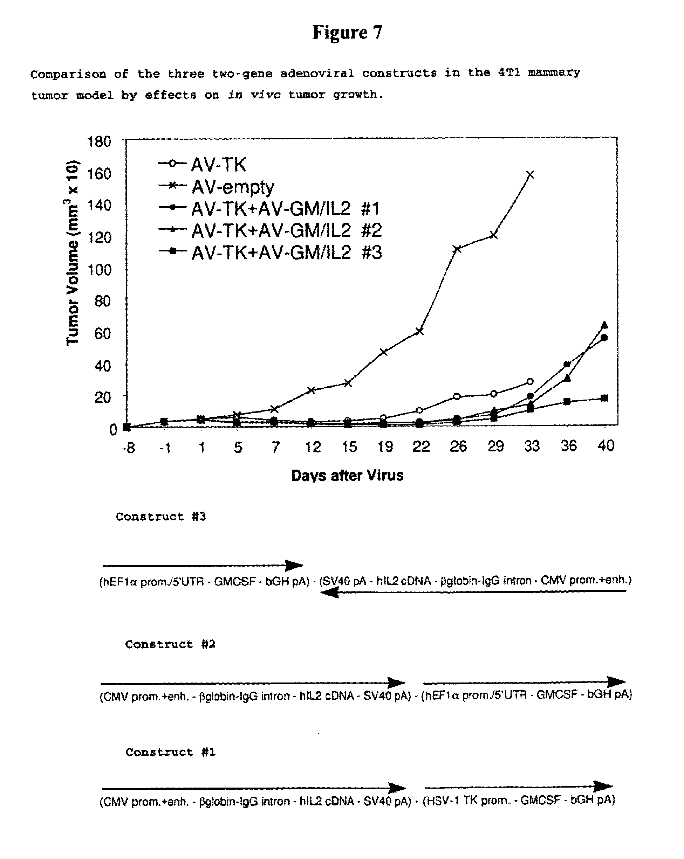

Figure 7 shows the effect of three different nucleic acid cassette constructs employed in an adenoviral vector system in combination with an additional TK-bearing adenovirus (Herpes Simplex Virus Thymidine Kinase, HSV-TK). The two control groups are labeled AV-empty (adenovirus bearing no exogenous transgene) and AV-TK (adenovirus bearing a TK gene). Construct #3, shown below the Table, has two polypeptide-encoding nucleic acids or DNAs oriented in opposite direction with respect to their reading frames. Constructs #1 and #2 are comparative examples, where all the reading frames are oriented in the same direction. The combination treatment of AV-TK plus AV-GM/IL2 in construct #3 (adenovirus bearing a TK gene in combination with an adenovirus of the invention bearing GM-CSF and IL-2) shows a marked reduction in tumor volume when compared to all the other treatments. Each group of animals, previously injected with 4T1 tumor cells, was treated with ganciclovir (GVC) for 10 days at 75 mg/kg body weight after administration of the indicated adenovirus.

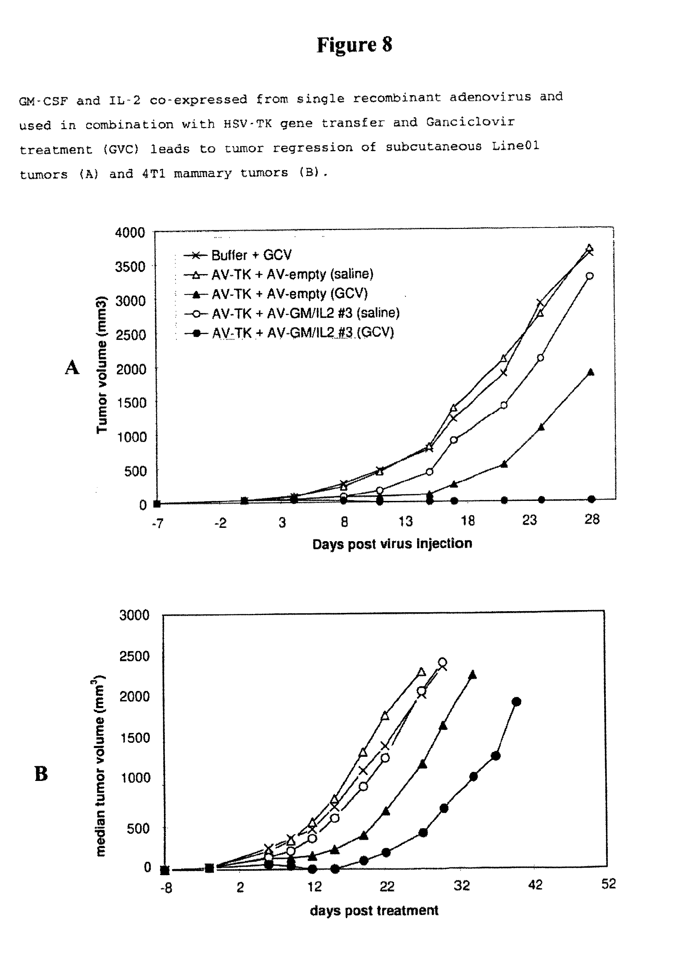

Figure 8 shows the same type of experiment as in Figure 7, which assesses the anti-tumor efficacy of the vectors and cassettes of the invention combined with a vector encoding HSV-TK in two murine non-immunogenic, spontaneously metastatic tumor models. The presence of the TK gene combined with treatment with ganciclovir is known to affect tumor volume. The combination with a vector of the invention, in this case encoding GM-CSF and IL-2 as in construct #3, provides particularly advantageous biological benefits in addition to the known biological effect of TK. In Figure 7, the construct #3 vector results in the greatest reduction of tumor volume. In the Figure 8 results, the construct #3 vector actually results in eradication of the Line01 tumor cells (Figure 8, panel A) and the greatest delay in 4T1 tumor growth (Figure 8, panel B).

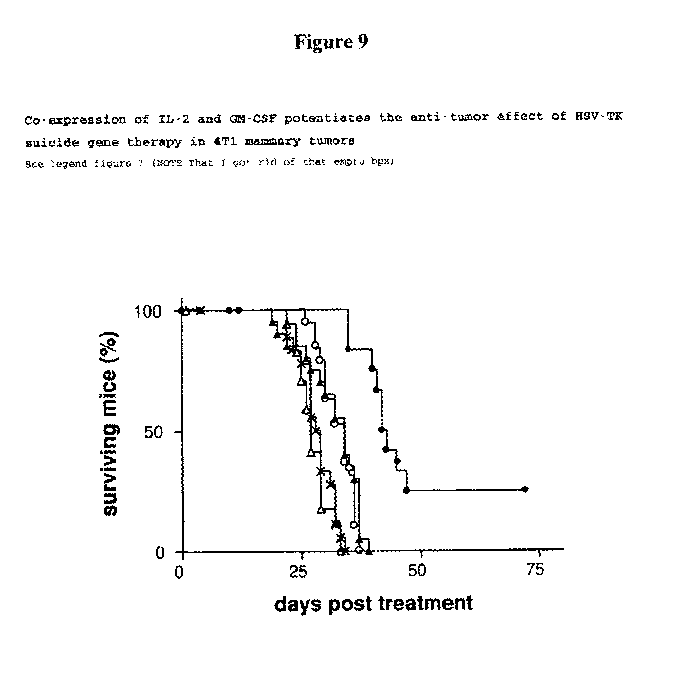

Figure 9 shows the same type of experiment as in Figure 8 with the 4T1 tumor cells, except the survival of the animal having the tumor cells is recorded. Again, the vector comprising the construct #3 in combination with the HSV-TK and ganciclovir treatment shows remarkably improved survival rates as compared to all other treatments. In panel B, the treatment regimen is as follows: day-8 subcutaneous tumor cell injection; day 0 adenoviral therapy via intratumoral administration; days 1-10 one injection per day of ganciclovir at 75 mg/kg; day 12 surgery to remove tumor. Panel B shows a dramatic reduction in metastasis for the course of the experiment.

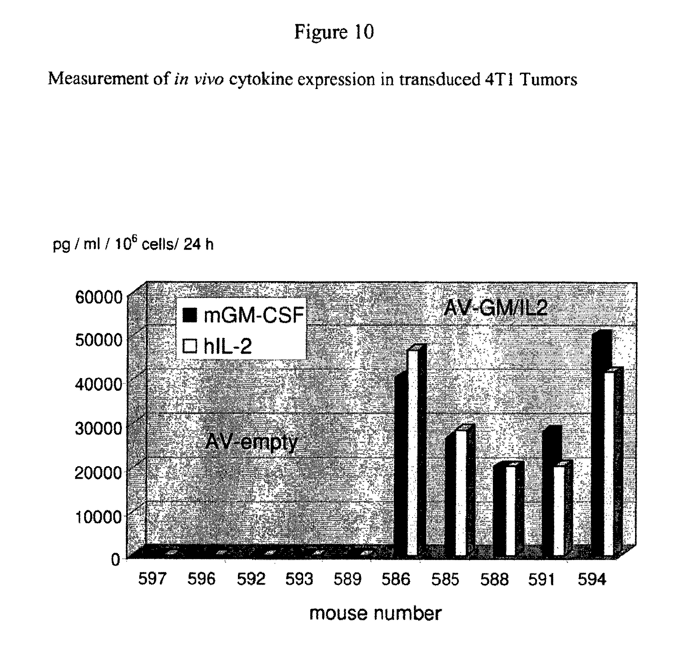

Figure 10 shows the *in vivo* expression levels of two cytokines 2 days after intratumoral administration of a control adenovirus (AV-empty) and a vector of the invention (AV-GM/IL2) into a mouse with 4T mammary tumor cells. Mice numbered 597, 596, 592, 593, and 589 were each administered the AV-empty virus *via* intratumoral injection. Mice numbered 586, 585, 588, 591, and 594 each were administered an adenovirus of the invention comprising mGM-CSF and hIL-2 coding regions arranged in opposite orientation with respect to their reading frames. The method results in very equal levels of mGM-CSF and hIL-2, as shown by the measurement of picograms of protein produced per ml of 10⁶ cells every 24 hours (pg/ml/10⁶ cells/24 hour). Protein levels can be determined by ELISA assay.

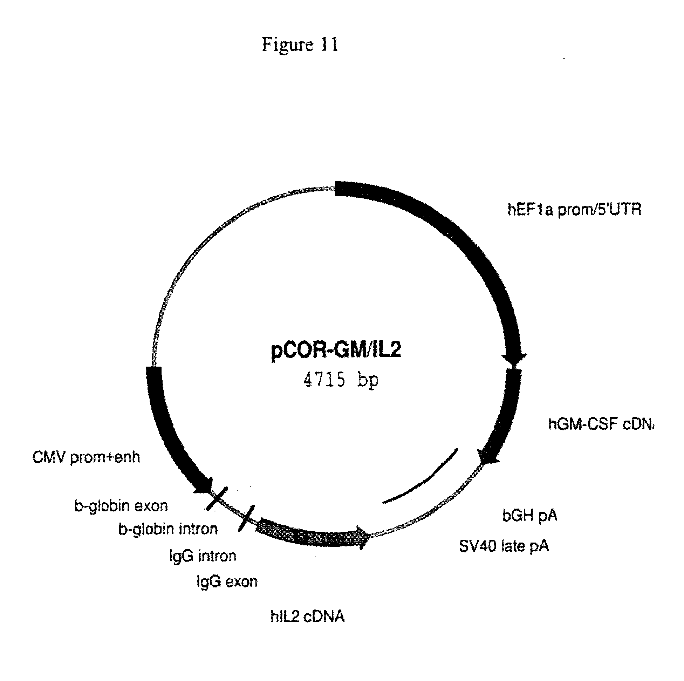

Figure 11 shows a map of a plasmid for expressing IL-2 and GM-CSF, and which can also be used in the production of an adenoviral vector containing an expression cassette of the invention. The arrows inside the plasmid indicate the orientation of the genetic elements. Elements of the plasmid are adapted from Soubrier *et al*., Gene Therapy 6:1482-88 (1999)(pCOR plasmids), however, any appropriate plasmid backbone for including an expression cassette can be selected. Combined, the b-globin exon and intron and IgG exon and intron sites noted make up an intron sequence that improves the expression levels of downstream linked protein or polypeptide encoding nucleic acid sequences. These sequences are noted as "intron sequences" in this disclosure. An intron sequence is also contained within the 5' UTR of the hEF1α sequences used here with the hGM-CSF gene sequence.

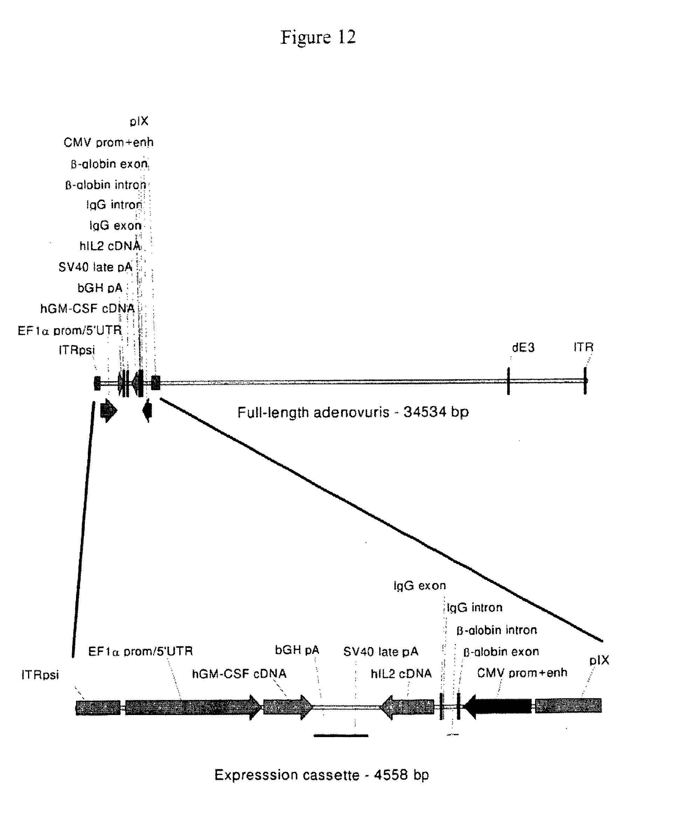

Figure 12 depicts a map of an exemplary recombinant adenovirus genome containing an expression cassette for expressing GM-CSF and IL-2, shown in detail below. The expression cassette is inserted into an E1 deletion in the Ad genome. Here, a deletion in the E3 region is represented by dE3.
Figure 13 depicts exemplary cassettes of the invention for expressing multiple transgenes (denoted "Tri 1" for the top and "Tri 2" for the bottom). Configurations for expressing a TK gene, an IL-2 gene, and a GM-CSF gene are shown. Two polypeptide coding regions are in opposite orientation with respect to their reading frames. An additional coding region or transgene, here, either IL-2 or TK, are also contained in the same cassette. An encephalomyocarditis virus (EMCV) IRES sequence is used here to combine the regulatory elements associated with one gene to express two genes. Other configurations, where a separate promoter/enhancer and poly A sites are used for the third or other transgenes, can also be selected for use.

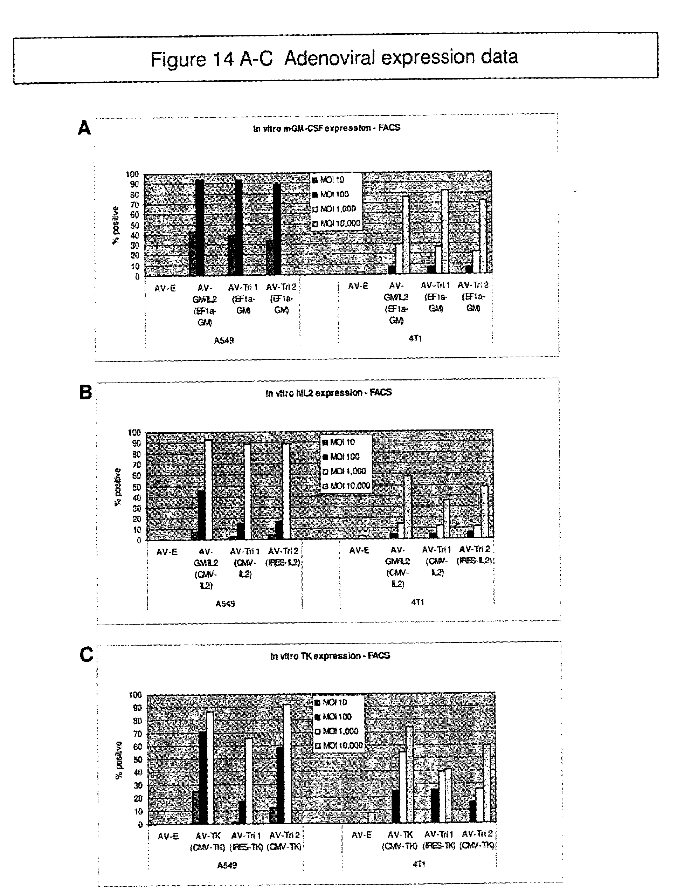

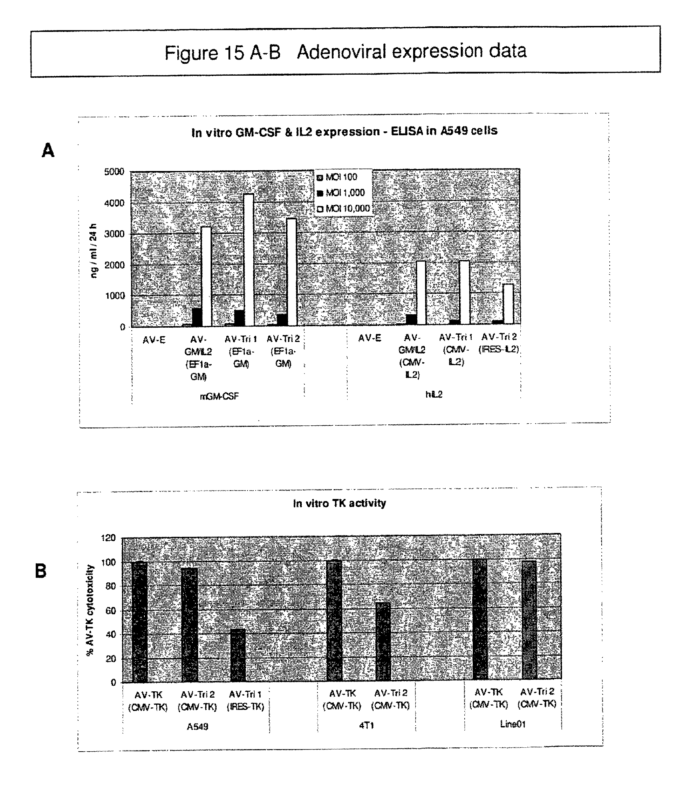

Figures 14 and 15 depict expression data for the two expression cassettes shown in Figure 13. The three-gene vector AV-Tri 1 is an adenoviral vector containing the cassette denoted Tri 1 in Figure 13, and the three-gene vector AV- Tri 2 adenoviral vector contains the cassette denoted Tri 2. The AV-E is an empty, control adenovirus containing no transgene. The adenoviral vector AV-TK is a one-gene vector, which contains the HSV-1 TK cDNA under the control of the CMV enhancer/promoter. The adenoviral vector AV-GM/IL2 is a two-transgene vector, such as that discussed for Figure 12. Figures 14 and 15 show expression data and comparison data at various numbers of viral particles per cell (MOI).

In Figure 14, the percent of infected cells that express enough of each transgene to be detected by FACS is depicted. Figure 14 top panel A shows the percentage of A549 cells and 4T1 cells expressing GM-CSF for some of the vector constructs and at several different MOIs. Similarly, middle panel B shows the percentage of IL-2 expressing cells. And, similarly, bottom panel C shows the percentage of TK expressing cells. The MOIs used were the following: MOI 10 & 100 for mGM-CSF in A549 cells; MOI 100, 1000, and 10,000 for mGM-CSF in 4T1 cells; MOI 10, 100, and 1000 for hIL2 and TK in A549 cells; MOI 100, 1000, and 10,000 for hIL2 and TK in 4T1 cells.

In Figure 15, the amount of cytokine secreted by infected A549 cells into their medium is depicted. Figure 15 A (left panel) shows the expression levels of mGM-CSF in A549 cells. The right panel of Figure 15 A shows the expression levels for hIL-2. A comparison of the protein levels from the left and right panels indicates that the three-gene vectors show the same relative equal levels of expression (30-80%) as the two-gene vector. The difference in protein expression levels between the GM-CSF and IL-2 transgenes is from about 2- to 3 ―fold.

Figure 15 B shows the result of TK activity on cells remaining in culture. Here, the more TK protein a particular virus produces, the more cytotoxic it is and, therefore, the fewer cells remain 5 days after infection. The relative cytotoxicity of the two-gene adenovectors compared to the monogene AV-TK adenovector is presented. The TK activity demonstrates that functionally active TK is being produced.

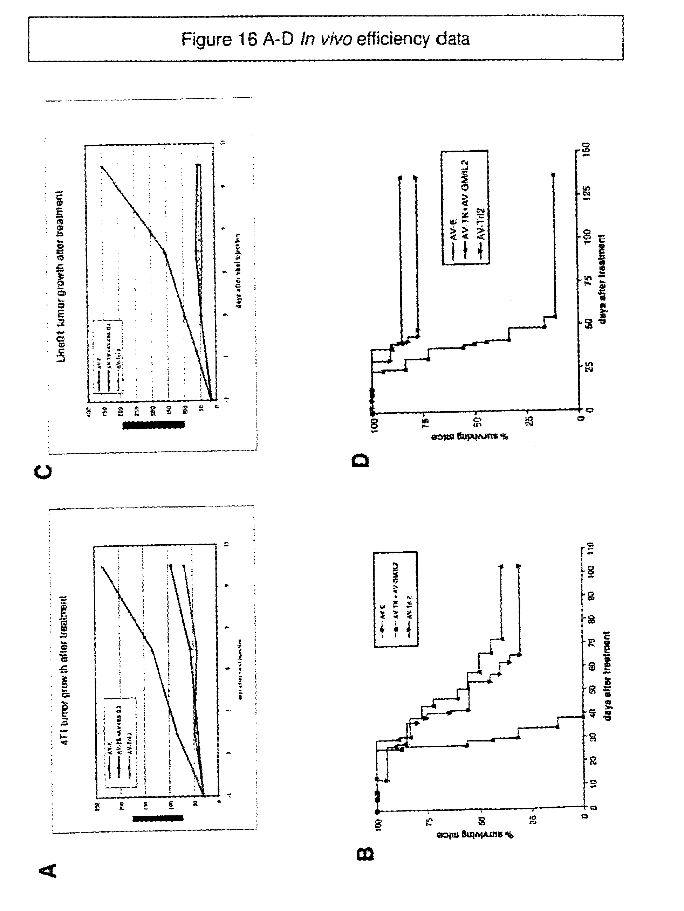

Figure 16 shows the *in vivo* function of the same vectors of Figures 14 and 15 in 4T1 and Line01 cells. Panel A demonstrates the dramatic and nearly instantaneous inhibition of tumor growth following treatment with the AV-TK + AV-GM/IL-2 cocktail in mice previously injected with tumor cells. An even further inhibition of tumor growth is shown in the three-gene adenoviral vector expressing HSV-TK, mGM-CSF, and hIL-2 (AV-Tri 2). Similarly, panel C shows the inhibition of tumor growth in Line01 tumor cells in mice. Panel B shows the survival of mice with 4T1 tumor cells after injection (here intratumoral injection) of the adenoviral vectors noted in Figure 14 and 15. Each of the vectors bearing cassettes of the invention leads to a marked increase in the survival rate compared to the negative control (AV-E). Panel D shows the same type of data as panel B, but in Line01 tumors.

### Detailed Description of Exemplary Embodiments

The embodiments and specific examples described below are merely exemplary of the scope and extent of the invention. One skilled in the art can select, construct, make, and use many different variations from the examples and embodiments described and still practice the invention or utilize one or more of the advantages provided by the teachings of this specification. The documents, scientific journal articles, texts, web pages, and other material referred to can be, each and every one, relied on or used to make and use aspects or variations of this invention. The following text does not repeat the fact that each and every reference cited is incorporated into this specification by reference, but we hereby do specifically incorporate each and every document, scientific journal article, text, web page, or other material referred to herein into the text of this specification by reference. As noted above, each and every reference cited can be relied on, in whole or in part, to make and use aspects of this invention. Additional or routine sources of information available to one skilled in the art can also be relied on and used.

As expression systems tax more and more specialized cells to produce increasingly complicated or specialized proteins, vectors with the ability to deliver complex genetic components into a broader range of appropriate cells become more of a necessity. The vectors, methods, and nucleic acids of the invention advantageously allow two or more proteins or polypeptides to be expressed at high, equal levels in a cell, through the use of only a single vector. For example, functional analysis of protein-encoding or polypeptide-encoding sequences often requires a convenient and flexible expression system. Because the functional analysis can often involve the expression of more than one protein, expression systems that utilize a single vector to express two or more polypeptides are extremely useful. For example, many reports have discussed synergies between angiostatin action and other bioactive proteins. *See, for example,* Yokoyama, *et al., Cancer Res.* 60:2190-6 (2000). A vector, cassette, or nucleic acid of the invention can be used to analyze the ability of a polypeptide to affect angiogenic pathways or functions in the presence of expressed, bioactive angiostatin. The apoptotic affect on various tumor cells is one specific example (discussed in Yokoyama, *et al*., above). Similarly, endostatin can be combined in a vector of the invention so that the vector causes the expression of endostatin and a second polypeptide (test polypeptide). Numerous other possibilities exist for the combination of a known bioactive protein and a second polypeptide (test polypeptide). Some of those possibilities are discussed below, but one skilled in the art can construct or devise many others.

Furthermore, the vectors, cassettes, nucleic acids, and methods of the invention can be used to combine two or more known bioactive polypeptides, as in the example of endostatin combined with angiostatin (Yokoyama, *et al., Cancer Res.* 60:2190-6 (2000)). Other examples include a combination of a cytokine with a drug sensitivity gene, such as IL-7 plus HSV-TK (thymidine kinase)(Sharma *et al., Gene Therapy* 4:1361-1370 (1997)), IL-18 plus CD (cytosine deaminase)(Ju *et al., Gene Therapy* 7:1672-1679 (2000), and tumor suppressor genes in combination with cytokines, such as p53 plus IL-2 (Putzer *et al., Hum. Gene Ther.* 9:707-718 (1998). Similarly, two cytokine genes can be combined or each of two chains of IL-12 can be combined. Many additional combinations can be selected and used with the present invention.

Some vectors for expressing multiple polypeptide-encoding sequences in mammalian cells have been noted. Components of these expression vectors and the expression systems and methods related to them can be used in the present invention without departing from the scope and intent. For example, a series of vectors named "pTRIDENT" has been discussed for including three separate coding regions and IRES I and IRES II sequences. *See* Fussenegger, *et al., Biotechnology and Bioengineering,* 57: 1-10 (1998) (noting ecdysone-responsive promoter, tet-regulatable promoter, SV40 promoter/enhancer, encepholamyocarditis virus translation enhancer, and SV40 polyadenylation signal). These vectors also discuss a β-lactamase-encoding sequence for ampicillin resistance as a bacterial marker, which is in a separate part of the vector. Of course, the pTRIDENT vectors require an IRES sequence and do not use sequences in opposite orientation. Both adenoviral and retroviral vectors dependent upon the encephalomyocarditis (EMCV) virus IRES sequences are discussed in He *et al., Gene* 175:121-125 (1996). Similarly, the adenovirus vector Ad5mIL-12 (*see* Greenberger, *et al., J. of Immunol.* 157:3006-3012 (1996)), is noted as containing the two chains of IL-12 on separate cassettes linked to cytomegalovirus immediate early promoters and SV40 poly A signals, each inserted into a separate region of the Ad5 genome (E1 and E3). And another adenovirus, AdCMV-IL-12 (*see* Siders, *et al., J. of Immunol.* 160: 5465-5474 (1998)), is noted as containing a CMV promoter. Finally, systems discussing bicistronic cytokine gene constructs, the tk-cytokine construct dependent upon a picornaviral IRES sequence (*see* Castleden, *et al., Human Gene Ther.* 8:2087-2102 (1997) and the lymphotactin-cytokine construct dependent upon the EMCV IRES (*see* Emtage, *et al., Human Gene Ther.* 10:697-709 (1999), have also been reported. The coding regions, polypeptide-encoding regions for bioactive compounds such as the cytokines and TK genes, the regulatory or control regions, and other aspect of these type of vector systems can be manipulated from the plasmids or other sources noted in these reports or elsewhere and adapted for use in the present invention.

The nucleic acids and cassettes of the invention can be used in a number of different vectors. The description below details some specific examples of viral vectors and plasmid vectors. However, the description should not be taken as a limitation of the scope of vectors that can be used in the invention.

### General Technology

In making and using aspects and embodiments of this invention, one skilled in the art may employ conventional techniques, such as molecular or cell biology, virology, microbiology, and recombinant DNA techniques. Exemplary techniques are explained fully in the literature. For example, one may rely on the following general texts to make and use the invention: Sambrook *et al., Molecular Cloning: A Laboratory Manual*, Second Edition (1989) Cold Spring Harbor Laboratory Press, Cold Spring Harbor, New York, and Sambrook *et al.* Third Edition (2001); *DNA Cloning: A Practical Approach,* Volumes I and II (D.N. Glover ed. 1985); *Oligonucleotide Synthesis* (M.J. Gaited. 1984); *Nucleic Acid Hybridization* (B.D. Hames & S.J. Higgins eds. (1985)); *Transcription And Translation* Hames & Higgins, eds. (1984); *Animal Cell Culture (RI.* Freshney, ed. (1986)); *Immobilized Cells And Enzymes* (*IRL* Press, (1986)); Gennaro et al. (eds.) *Remington's Pharmaceutical Sciences,* 18th edition; B. Perbal, *A Practical Guide To Molecular Cloning* (1984); F.M. Ausubel et al. (eds.), *Current Protocols in Molecular Biology,* John Wiley & Sons, Inc.(updates through 2001), Coligan et al. (eds.), *Current Protocols in Immunology,* John Wiley & Sons, Inc.(updates through 2001); W. Paul et al. (eds.) *Fundamental Immunology,* Raven Press; E.J. Murray *et al.* (ed.) Methods in Molecular Biology: Gene Transfer and Expression Protocols, The Humana Press Inc. (1991)(especially vol.7); and J.E. Celis *et al., Cell Biology: A Laboratory Handbook*, Academic Press (1994).

As used herein, a "vector" means any nucleic acid or nucleic acid-bearing particle, cell, or organism capable of being used to transfer a nucleic acid into a host cell. The term "vector" includes both viral and nonviral products and means for introducing the nucleic acid into a cell. A "vector" can be used *in vitro, ex vivo*, or *in vivo.* Non-viral vectors include plasmids, cosmids, and can comprise liposomes, electrically charged lipids (cytofectins), DNA-protein complexes, and biopolymers, for example. Viral vectors include retroviruses, lentiviruses, adeno-associated virus, pox viruses, baculovirus, reoviruses, vaccinia viruses, herpes simplex viruses, Epstein-Barr viruses, and adenovirus vectors, for example. Vectors can also comprise the entire genome sequence or recombinant genome sequence of a virus. A vector can also comprise a portion of the genome that comprises the functional sequences for production of a virus capable of infecting, entering, or being introduced to a cell to deliver nucleic acid therein.

"Regulatory" or "control" sequences or "regulatory" or "control" regions are nucleic acid sequences that regulate the expression of a second nucleic acid sequence. A regulatory or control sequence may include sequences that are, in nature, responsible for expressing a particular nucleic acid (a homologous sequence or region) or may include other sequences, such as heterologous, synthetic, or partially synthetic sequences. In particular, the sequences can be of eukaryotic or viral origin that stimulate or repress transcription of a gene in a specific or non-specific manner and in an inducible or non-inducible manner. Regulatory or control regions include origins of replication, RNA splice sites, introns, chimeric or hybrid introns, promoters, enhancers, transcriptional termination sequences, poly A sites, locus control regions, signal sequences that direct the polypeptide into the secretory pathways of the target cell, and introns. A "heterologous" regulatory region is not naturally associated with the expressed nucleic acid it is linked to. Included among the heterologous regulatory regions are regulatory regions from a different species, regulatory regions from a different gene, hybrid regulatory sequences, and regulatory sequences that do not occur in nature, but which are designed by one of ordinary skill in the art. "Operably linked" refers to an arrangement of sequences or regions wherein the components are configured so as to perform their usual or intended function. Thus, a regulatory or control sequence operably linked to a coding sequence is capable of effecting the expression of the coding sequence. The regulatory or control sequences need not be contiguous with the coding sequence, so long as they function to direct the proper expression or polypeptide production. Thus, for example, intervening untranslated but transcribed sequences can be present between a promoter sequence and the coding sequence and the promoter sequence can still be considered "operably linked" to the coding sequence.

One of skill in the art is familiar with the selection or optimization of regulatory or control regions for specific purposes or applications. *(See,* for example, Yew *et al*., Hum. Gene Ther. 8:575-84 (1997).) In the case of this invention, the high levels of transgene expression are typically achieved through selection of regions that produce or result in high expression, using promoters know to be highly active. Thus, promoter or promoter/enhancer combinations of the same relative efficiency in a cell can be selected for expressing two transgenes at the same relative level. The same can be said of the intron sequences, poly A sites, consensus translation start site sequences, and other sites that may be included in an expression cassette. (*See,* for example, Yew *et al.,* Hum. Gene Ther. 8:575-84 (1997); Kozak, Cell 41:283-292 (1986); Brinster, *et al*., PNAS 85:836 (1988); Jackson, *et al*., Cell 62:15 (1990); Kozak, *et al.* Mol. Cell. Biol. 9:5134 (1989); and *Current Protocols in Molecular Biology.)* One of skill in the art is familiar with selecting and testing various regions in order to optimize the expression for certain applications. The use of the instant invention in allowing new and improved combinations and orientations of regulatory and control regions increases both the flexibility and performance of an expression cassette and the vectors and expression systems that contain them. Both the high levels and the stable and relatively equal levels of expression using the expression cassettes of the invention form advantageous aspect for incorporation into any desired vector.

A "cassette" refers to a segment of DNA or nucleic acid that can be inserted into a vector at desired or specific sites. Ideally, the specific sites are defined by restriction sites so that the cassettes can easily be dropped into the vector and so that the cassette is inserted in the proper reading frame and orientation for transcription and translation. However, a cassette can be inserted by blunt-end ligation or other methods, such as homologous recombination, that do not depend on restriction sites or restriction enzyme cleavage. The segment of DNA or nucleic acid that comprises the cassette encodes at least one polypeptide, gene, or protein of interest. An "expression cassette" is a cassette containing sequences that direct or that are capable of generating at least one transcript or polypeptide in an appropriate host cell system or *in vitro* system.

A cell has been "transfected" by exogenous or heterologous DNA when the DNA has been introduced inside the cell. A cell has been "transformed" or "transduced" by exogenous or heterologous DNA when the transfected DNA effects a phenotypic change or detectable modification in the cell.

A "nucleic acid" (NA) is a polymeric compound comprised of covalently linked nucleotides, from whatever source. Nucleic acid includes polyribonucleic acid (RNA) and polydeoxyribonucleic acid (DNA), both of which may be single-stranded or double-stranded. DNA includes cDNA, genomic DNA, synthetic DNA, and semi-synthetic DNA. The term "nucleic acid" also captures sequences that include any of the known base analogues of DNA and RNA.

A "recombinant DNA molecule" is a DNA molecule that has undergone at least one molecular biological manipulation, as known in the art. Typically, this manipulation occurs *in vitro* but it can also occur within a cell, as with homologous recombination. A DNA "coding sequence" is a sequence capable of being transcribed and translated into a polypeptide in a cell *in vitro* or *in vivo* when placed under the control of appropriate regulatory sequences. The boundaries of the coding sequence are determined by a start codon at the 5'(amino) terminus and a translation stop codon at the 3'(carboxyl) terminus. A polyadenylation signal and transcription termination sequence will usually be located 3' to the coding sequence. In eukaryotic cells, poly A sites or polyadenylation signals are control sequences.

A "promoter sequence" is a DNA regulatory region capable of binding RNA polymerase in a cell and/or capable of initiating transcription of a downstream (3' direction) coding sequence. Unless otherwise indicated, the promoter sequence is bounded at its 3' terminus by the transcription initiation site and extends upstream (5' direction) to include the minimum number of bases necessary to initiate transcription above background levels. Preferably, a strong promoter is linked 5' to the transgene so as to drive transgene expression in a variety of cell types. For example, a strong promoter particularly suited for use is the cytomegalovirus (CMV) promoter. By the term "CMV promoter" is meant a promoter existing naturally in or derived from a CMV strain having a DNA sequence controlling transcription of the immediate early (IE) gene of CMV. The CMV promoter is available through the plasmid pCMVβ (GenBank Acc. No. U02451). Alternatively, other strong eukaryotic promoters are suitable, including a hybrid promoter such as a CMV/Ela hybrid promoter. A coding sequence is "under the control" of transcriptional and translational control sequences in a cell when RNA polymerase transcribes the coding sequence into mRNA, which is then optionally trans-RNA spliced and translated into the protein encoded by the coding sequence. A "signal sequence" is included at the beginning of a coding sequence of a protein to be expressed on the surface of a cell or secreted from the cell. Signal sequences can be found associated with a variety of proteins native to eukaryotes and prokaryotes.

As used here, a "transgene" is a nucleic acid molecule having a sequence from which a functional or active transcript can be produced in a cell or other transcription system, including a complementary nucleic acid, a DNA, or RNA version of the nucleic acid molecule or the complement. There are numerous transgenes noted throughout this disclosure, but the possible transgene that can be selected for use in the invention are not limited to those specifically mentioned or listed. Preferred transgenes include cytokines, tumor suppressing genes, cytostatic or cell cycle arresting genes, and those that have immunomodulatory or cancer specific activities or biological effects, as well as those that encode enzymatic activity that converts a compound into an active drug or converts a pro-drug into an active drug. Specific, non-limiting examples include genes sequences encoding a particular protein, such as IL-2, IL-12, α- interferon, γ- interferon, HSV thymidine kinase (TK) or other TK, GM-CSF, G-CSF, M-CSF, tumor necrosis factor α or β, an interleukin gene, such as IL-1, IL-2, IL-3, IL-4, IL-5, IL-6, IL-10, IL-11, IL-12, IL-15, IL-18, IL-18, IL-20, an interferon, such as IFN α, β, or γ subtypes such as α-2b, and interferon fusions such as α-2α-1, chemokines, such as MGSA (melanoma growth stimulatory activity protein), PBP, MIP2, Mig, PBSF (pre B cell growth stimulating factor), MCP (monocyte chemotactic protein), MIP (macrophage inflammatory protein), angiostatin, endostatin, an amino-terminal fragment of plasminogen having an amino acid sequence of plasminogen from about amino acid residue 1 to about residue 333, anti-angiogenic fragment of plasminogen or angiostatin or endostatin or thrombospondin, such as thrombospondin 1, inhibitors of VEGF such as Tie2, or platelet factor IV, FGF, aFGF, bFGF, VEGF, or an angiogenic fragment of FGF, aFGF, bFGF, VEGF, or thrombospondin, a thymidine kinase or cytosine deaminase activity-possessing polypeptide, a serine/threonine kinase Akt activity, Akt-1, Akt-2, Akt-3, p53, Rb, mda-7, rap 1A, DCC, k-rev2, k-rev3, p21, E2F-Rb, cyclin dependent kinase inhibitors, such as p16, p15, p18, p19, growth arrest homoebox genes, such as GAX, antigenic genes or nucleic acids encoding an antigenic fragment of polypeptide, antibody-encoding genes or epitope-binding genes, such as scFv and scFv-containing fragments and polypeptides, and adenosine deaminase. Additional proteins or fragments are noted here or can be selected from many known in the art (including those listed in WO 98/37185) or listed in genomic or other sequence databases. Alternatively, a transgene may have a sequence that produces a functional transcript, such as with anti-sense sequences like those designed to bind mRNA or DNA in a cell to prevent transcription or translation, a ribozyme, an aptamer, or other active RNA molecules or chimeric nucleic acids. As noted, the expression cassettes and vectors of the invention may comprise, two, three, or even more transgenes. Preferably, they contain two or three transgenes.

The "intron sequences," "intron" regulatory elements, or "chimeric introns" noted here can be any untranslated nucleic acid sequence, such as those found between the promoter and the beginning of the coding sequence, that functions to enhance the stability of mRNA transcripts, facilitate the transport of mRNA into the cytoplasm, or improve expression levels. A number of intron sequences known for these functions have been used in the art (Buchman *et al.* Molec. Cell. Biol. 8: 4395-4405 (1988)), such as intron A from CMV immediate early gene (CMV IE1), rabbit beta-1 globin intron, rabbit beta-globulin intron II, human beta-globin first intron, thymidine kinase-derived intron sequences, human immunoglobulin gene intron, and a number of hybrid or chimeric intron sequences. Typically, a donor site, a branchpoint site, and an acceptor site will be included in the intron sequence.

The term "corresponding to" is used herein to refer to similar or homologous sequences, whether the exact position is identical or different from the molecule to which the similarity or homology is measured. A nucleic acid or amino acid sequence alignment may include spaces. Thus, the term "corresponding to" refers to the sequence similarity, and not the numbering of the amino acid residues or nucleotide bases. "Percent identity" between two nucleic acids or two polypeptide molecules refers to the percent defined by a comparison using a tblastx, blastx, blastn, or blastp search at the default settings *(see, for example*, NCBI BLAST home page: http://www.ncbi.nlm.nih.gov/BLAST/). "Homology" can be determined by a direct comparison of the sequence information between two polypeptide molecules by aligning the sequence information and using readily available computer programs. Alternatively, homology can be determined by hybridization of polynucleotides under conditions allowing for the formation of stable duplexes between homologous regions and determining of identifying double-stranded nucleic acid.

A "functional homologue" or a "functional equivalent" of a given polypeptide or sequence includes molecules derived from the native polypeptide sequence, as well as recombinantly produced or chemically synthesized polypeptides, which function in a manner similar to the reference molecule or achieve a similar desired result. Thus, a "functional homologue" or a "functional equivalent" of a given adenoviral nucleotide region includes similar regions derived from a different adenovirus serotype, nucleotide regions derived from another virus, or from a cellular source, as well as recombinantly produced or chemically synthesized nucleic acids that function in a manner similar to the reference nucleic acid region in achieving a desired result, such as a result in a particular assay or cell phenotype. So, for example, a functional homologue of AAV Rep encompasses derivatives and analogues including any single or multiple amino acid additions, substitutions and/or deletions occurring internally or at the amino or carboxy termini thereof, so long as integration activity remains. And, functionally equivalent adenovirus sequences of different sizes can be used on a pro-viral plasmid so that the sequences each result in the generation of a recombinant adenovirus that possesses the same capabilities to express polypeptides. Functional homologues of bioactive polypeptide-encoding sequences include mutations and substitutions that do not substantially effect a biological property of the polypeptide. Thus, mutations or substitutions that result in silent mutations, conservative amino acid substitutions, alterations in post-translation processing, or deletions or additions of nucleotides or amino acids that do not substantially alter a biological function or activity can be made to the sequences used in the invention. Numerous methods for creating and testing mutation-containing sequences are known in the art and can be used.

One or more amino acid residues within a sequence can be substituted by another amino acid of a similar polarity, which acts as a functional equivalent when the substitution results in no significant change in activity in at least one selected biological activity or function. Substitutions for an amino acid within the sequence may be selected from other members of the class to which the amino acid belongs. For example, the nonpolar (hydrophobic) amino acids include alanine, leucine, isoleucine, valine, proline, phenylalanine, tryptophan and methionine. Amino acids containing aromatic ring structures are phenylalanine, tryptophan, and tyrosine. The polar neutral amino acids include glycine, serine, threonine, cysteine, tyrosine, asparagine, and glutamine. The positively charged (basic) amino acids include arginine, lysine and histidine. The negatively charged (acidic) amino acids include aspartic acid and glutamic acid. These alterations will not affect apparent molecular weight as determined by polyacrylamide gel electrophoresis, or significantly affect the isoelectric point. When a transgene used encodes a functional transcript, such as an anti-sense- producing sequence, a ribozyme, or an aptamer, a functional equivalent includes sequences that perform substantially the same function but differ in nucleotide sequence. Thus, the transgenes used here need not be protein-encoding sequences.

"Isolated," when referring to a nucleic acid sequence or vector, means that the indicated molecule is present in the substantial absence of other biological macromolecules of the same type. Thus, an "isolated nucleic acid molecule that encodes a particular polypeptide" refers to a nucleic acid molecule substantially free of other nucleic acid molecules that do not encode the particular polypeptide. However, the preparation or sample containing the molecule may include other components of different types. In addition, "isolated from" a particular molecule may also mean that a particular molecule is substantially absent from a preparation or sample.

### Exemplary Gene Transfer Vectors

As discussed above, a "vector" is any nucleic acid or nucleic acid-bearing composition, particle, or cell capable of being transferred into a host cell. A "vector" can be, thus, a plasmid, cosmid, BAC, YAC, virus, bacteriophage, or fragment thereof. Preferred vectors are viral-derived vectors, such as retroviruses, herpes viruses, reoviruses, oncolytic viruses, lentiviruses, adenoviruses, and adeno-associated viruses. The viral vector can be replication-competent, can replicate only under certain conditions, or be replication-defective. Expression from a vector in cultured cells, targeted tissues, or organisms can be effected by targeting the vector to specific cells, such as with a viral vector or a receptor ligand, or by using a tissue-specific promoter, or by using a specific administration method, or a combination of these. Viral vectors commonly used for *in vivo* or *ex vivo* targeting and gene transfer procedures are DNA-based vectors, reoviruses, and retroviral vectors. Methods for constructing and using viral vectors are known in the art (*see, e.g.,* Miller and Rosman, *BioTechniques* 7:980-990 (1992)).

DNA viral vectors include an attenuated or defective DNA virus, such as but not limited to herpes simplex virus (HSV), papillomavirus, Epstein-Barr virus (EBV), adenovirus (Ad), adeno-associated virus (AAV), and the like. Defective viruses, which entirely or partially lack certain viral genes, are preferred. In certain circumstances, the viral vectors are replication-defective, that is, they are unable to replicate autonomously in the target cell. In general, the genome of the replication-defective viral vectors used within the scope of the invention lack at least one region necessary for the replication of the virus in the infected cell. These regions can either be eliminated (in whole or in part) or be rendered non-functional by any technique known to a person skilled in the art. These techniques include deletion (complete or partial), substitution (by other sequences, in particular by inserted nucleic acid), partial deletion, or addition of one or more bases to an essential region. These techniques may be performed *in vitro* (on the isolated DNA) or *in situ,* using, for example, the techniques of genetic manipulation, homologous recombination, or by treatment with mutagenic agents. Preferably, the replication-defective virus retains the sequences of its genome necessary for encapsulating the viral particles.

Use of defective viral vectors or conditionally replicative viral vectors allows for administration to cells in a specific, localized area, without concern that the vector can infect or spread to other cells. Thus, a specific tissue can be specifically targeted. Examples of particular vectors include, but are not limited to, a defective herpes virus 1 (HSV1) vector (Kaplitt et al., *Molec. Cell. Neurosci.* 2:320-330 (1991)); defective herpes virus vector lacking a glycoprotein L gene, or other defective herpes virus vectors (WO 92/05263); an attenuated adenovirus vector, such as the vector described by Stratford-Perricaudet et al. (*J. Clin. Invest.* 90:626-630 (1992); *see also* La Salle et al., *Science* 259:988-990 (1993)); and a defective adeno-associated virus vector (Samulski et al., *J. Virol.* 61:3096-3101(1987); Samulski et al., *J. Virol* 63:3822-3828 (1989); Lebkowski et al., *Mol. Cell. Biol.* 8:3988-3996 (1988)).

*Adenovirus vectors.* Adenoviruses are a family of double-stranded DNA viruses that can infect both dividing and non-dividing cells (Verma and Somia, *Nature* 389: 239-242 (1997)). They have a genome of 36 kb, containing over a dozen genes. Vectors based on this virus have received the most attention for delivery of genes into mammalian cells (Fasbender *et al., J. Biol. Chem.* 272: 6479-6489 (1997); Qian *et al.* Circ. Res. 88: 911-917 (2001)). Recombinant adenoviruses display many advantages for use as transgene expression systems, including the tropism for both dividing and non-dividing cells, minimal pathogenic potential, ability to replicate to high titer for preparation of vector stocks, and the potential to carry large inserts *(see e.g.,* Berkner, K. L., *Curr. Top. Micro. Immunol*., 158:39-66 (1992); Jolly D., *Cancer Gene Therapy,* 1:51-64 (1994)). Adenovirus vectors can accommodate transgenes as large as eight (8) kb by deleting regions of the genome dispensable for growth, such as the E3 region. Cell lines exist that supply non-dispensable adenovirus gene products in trans (e.g., E1, E2a, E4). This allows a variety of transgenes to be inserted into the adenovirus genome (see e.g. Graham, F. L., *J. Gen. Virol.,* 36:59-72 (1977); Imler et al., *Gene Therapy*, 3:75-84 (1996)). In a preferred embodiment, the vector of the invention is a recombinant adenovirus vector. A particularly preferred embodiment is a recombinant, replication-defective adenovirus vector. These vectors can be particularly effective for delivery of angiogenesis factors, such as angiogenesis inhibitors angiostatin and endostatin, or cytokine genes, such as GM-CSF, IL-2, or a number of other interleukins, or the delivery of any combination of bioactive proteins or polypeptides.

Various serotypes of adenovirus exist and can be used (see ATCC Catalogue of Animal Viruses). Of these serotypes, preferred are type 2 or type 5 human adenoviruses (Ad 2 or Ad 5) or adenoviruses of animal origin (CAV1 or CAV2) (see W0 94/26914). Those adenoviruses of animal origin, which can be used within the scope of the present invention, include adenoviruses of canine, bovine, murine, ovine, porcine, avian, and simian origin. Preferably, the adenovirus of animal origin is a canine adenovirus, more preferably a CAV2 adenovirus (e.g., Manhattan or A26/61 strain (ATCC VR-800), for example). The genome of the adenovirus Ad5 has been sequenced in its entirety and is available from databases (see, in particular, Genbank M73260). Similarly, parts, if not the whole, of other adenoviral genomes have also been sequenced.

Preferably, the replication-defective recombinant adenoviral vectors of the invention comprise the ITRs, an encapsidation sequence, and nucleic acids for expressing multiple transgenes or polypeptides. Still more preferably, at least the E1 region of the adenoviral vector is non-functional. Many different specific deletions in the E1 region can render a virus non-functional or conditionally non-functional or even defective in E1 and are known in the art, but a few examples are Ad5 deletions extending from nucleotides 455 to 3329 (PvuII-BglII fragment), or 382 to 3446 (Hinfll-Sau3A fragment), or 382 to 3513. Some other examples of El-deleted adenoviruses are disclosed in EP 185,573, WO 00/29573, WO 96/10642, WO 96/5506, WO 00/24408, and Nunes *et al*., *Human Gene Therapy* 10:2515-26 (1999).

Other Ad regions may also be modified, in particular the E3 region (W095/02697), the E2 region (W094/28938), the E4 region (W0 94/28152; FR 9413355; W0 94/12649; and W0 95/02697), or in any of the late genes *L1-L5*. In another preferred embodiment, the adenoviral vector has a deletion in the E1 and E3 regions or E1 and E4 regions. Examples of E1/E3-deleted adenoviruses are disclosed in Bett *et al, PNAS* 91:8802-8800 (1994), for example. Examples of E1/E4-deleted adenoviruses are disclosed in, for example, Miyake *et al., PNAS* 93:1320-24 (1996); W0 95/02697; and W096/22378. E4 deletions may encompass the ORF3 and ORF6 reading frames, for example, as well as the entire E4 region. In still another preferred embodiment, the adenoviral vector has a deletion in the E1 region into which the E4 region and the nucleic acid sequence are inserted *(see* WO 94/12649 and FR 94 13355). In addition, minimal-adenovirus vectors can be used, as discussed in, for example, Zhang *et al., Thromb. Haemost*. 82:562-571 (1999).

The replication defective recombinant adenoviruses according to the invention can be prepared by any technique known to the person skilled in the art (for example, the techniques noted in the references herein and Levrero et al., *Gene* 101:195 (1991), Gosh-Choudhury et al., *Gene* 50:161 (1986), EP 185 573; Graham, *EMBO J.* 3:2917 (1984)). In particular, they can be prepared by homologous recombination between an adenovirus and a plasmid carrying, *inter alia,* the DNA sequence of interest. The homologous recombination is effected following cotransfection of the adenovirus and plasmid into an appropriate cell line. The cell line employed should preferably (i) be capable of being transformed by the plasmid and adenovirus DNA, and (ii) contain the sequences which complement the part of the genome of the replication defective adenovirus, preferably in integrated form), and preferably with little to no homology to adenoviral sequences contained in the recombinant shuttle vector in order to avoid recombination and production of replication competent adenovirus (RCA). Examples of cell lines to be used are the human embryonic kidney cell line 293 (Graham *et al., J. Gen. Virol.* 36:59-72 (1977)), which contains the left-hand portion of the genome of an Ad5 adenovirus (12%) integrated into its genome, PER.C6 cells, which contain the E1 region *(see, for example*, WO 97/00326, Fallaux *et al*., Hum Gen. Ther. 9: 1909-1917 (1998)) and cell lines able to complement the E1 and E4 functions, as described in W0 94/26914 and W0 95/02697, and Fallaux *et al*, Hum Gen. Ther. 7: 215-222 (1996).

The following table lists additional examples and methods.

| **Adeno genome contained** **in** | **Transgene cassette** **contained in** | **Recombination** **carried out in** | **Reference** |
|---|---|---|---|
| | | | |
| restricted Ad DNA | Plasmid | 293 | Graham and Prevec, Biotechnology 20:363-90 (1992) |
| | | | |
| Plasmid | Plasmid | 293 | Bett et al. (1994) PNAS 91:8802-6 |
| | | | |
| cosmid + restricted Ad DNA | Cosmid | 293 | Miyake et al. (1996) PNAS 93:1320-4 |
| | | | |
| YAC | Plasmid | Yeast | Ketner et al. (1994) PNAS 91:6186-90 |
| | | | |
| Plasmid | isolated DNA fragment | Bacteria | Chartier et al. (1996) Virol. 70:4805-10 |
| | | | |
| Plasmid | isolated DNA fragment | Bacteria (not recombination but direct cloning) | Souza & Armentano (1999) Biotechniques 26:502-8 |
| | | | |
| Plasmid | Plasmid | Bacteria (not recombination but direct cloning) | Danthinne (2001) Biotechniques 30:612-6, 618-9 |
| | | | |
| Plasmid (adenoviral backbone plasmid) | Plasmid (suicide shuttle plasmid) | Bacteria | Crouzet et al. (1997) PNAS 94:1414-19 |
| | | | |

Recombinant adenoviruses are recovered and purified using standard molecular biological and virology techniques, which are well known to one of ordinary skill in the art.

Another class of adenoviral vectors that can be selected for use are the conditional replicative recombinant vectors. These vectors are designed to replicate only in certain cells. For example, cells that are deficient in p53 or Rb function can support the replication of adenoviruses that have been mutated or deleted in particular regions of the genome (*see, for example*, U.S Patents 6,111,243, 5,972,706, and published PCT documents WO 00136650, WO 0024408). Typically, these adenoviruses contain mutations or modifications in the E1a or E1b region of the adenoviral genome. The mutation or modication alters the ability of the virus to replicate within cells that contain certain mutated proteins. For those viruses designed to replicate in tumor cells, it is generally an oncoprotein or other tumor-related or growth-related cell protein that contains a mutation.

*Adeno-associated viruses.* The adeno-associated viruses (AAV) are single-stranded DNA viruses of relatively small size that can integrate, in a stable and site-specific manner, into the genome of the cells that they infect. They are able to infect a wide spectrum of cells without inducing any effects on cellular growth, morphology or differentiation, and they do not appear to be involved in human pathologies. The AAV genome has been cloned, sequenced and characterized. It encompasses approximately 4700 bases and contains an inverted terminal repeat (ITR) region of approximately 145 bases at each end, which serves as an origin of replication for the virus. The remainder of the genome is divided into two essential regions which carry the encapsidation functions: the left-hand part of the genome, which contains the rep gene involved in viral replication and expression of the viral genes; and the right-hand part of the genome, which contains the cap gene encoding the capsid proteins of the virus.

The use of vectors derived from the AAVs for transferring genes in *vitro* and *in vivo* has also been described (see WO 91/18088; WO 93/09239; US 5,952,221, US 4,797,368, US 5,139,941, US 6,027,931, EP 488 528). These publications describe various AAV-derived constructs in which the rep and/or cap genes are deleted and replaced by a gene of interest, and the use of these constructs for transferring a gene of interest *in vitro* or *in vivo*. The replication-defective recombinant AAVs can be prepared by cotransfecting a plasmid containing the nucleic acid sequence of interest flanked by two AAV inverted terminal repeat (ITR) regions, and a plasmid carrying the AAV encapsidation genes (rep and cap genes), into a cell line which is infected with a human helper virus (for example an adenovirus). The AAV recombinants produced can be purified by standard techniques.

The invention also relates, therefore, to an AAV-derived recombinant virus whose genome comprises a nucleic acid or expression cassette of the invention, flanked by the AAV ITRs. The invention also relates to a plasmid comprising a nucleic acid or expression cassette of the invention flanked by two ITRs from an AAV. The plasmid can be used for transferring the nucleic acid or expression cassette, as in the case of a pseudo-virus, the use and construction of which is known in the art. AAV vectors can be constructed using techniques well known in the art. *See, e.g.,* US 5,173,414; WO 91/18088; WO 93/09239; US 5,952,221; US 4,797,368; US 5,139,941; US 6,027,931; EP 488 528; WO 92/01070; WO 93/03769; Lebkowski *et al., Molec. Cell. Biol.* 8:3988-3996 (1988); Vincent *et al.* Vaccines 90, Cold Spring Harbor Laboratory Press, (1990); Carter, B. J. *Current Opinion in Biotechnology* 3:533-539 (1992); Paul *et al., Cancer Gene Ther.* 7:308-15 (2000); Muzyczka, N. *Current Topics in Microbiol. and Immunol.* 158:97-129 (1992); Kotin, R. M. *Human Gene Therapy* 5:793-801 (1994); Shelling and Smith (1994) *Gene Therapy* 1:165-169 (1994); and Zhou *et al. J. Exp. Med.* 179:1867-1875 (1994).

Selected adenoviral genes or gene regions (e.g., E1a, E1b, E2a, E4 and VA RNA), or functional homologues thereof, can be excised from a viral genome, or from a vector containing the same, and inserted into a suitable vector either individually, or linked together, to provide an AAV accessory function construct using standard ligation techniques such as those described in Sambrook *et al., Molecular Cloning: A laboratory Manual,* Third Edition (2001). One such construct can be engineered to include four nucleic acid molecules derived from the Ad-5 genome: a VA RNA-containing region; an E2a-containing region; an E4-containing region and an E1a, E1b-containing region. Specifically, a 1,724 bp SalI-HinDIII VA RNA-containing fragment (about 9,831 to about 11,555 of Ad-2 genome); a 5,962 bp SrEI-BamHI E2a-containing fragment (about 21,606 to about 27,568 of Ad-2 genome); a 3,669 bp HphI-HinDIII E4-containing fragment (about 32,172 to about 36,841 of the Ad-2 genome); and a 4,102 bp BsrGI-Eco47III E1a-, E1b-containing fragment (about 192 to about 4294 of Ad-2 genome), wherein the nucleic acid molecules are ligated together to provide a complete complement of accessory functions in a single construct or nucleic acid. Conventional ligation reactions can be used to generate the constructs or plasmids containing them. The assembled AAV construct can then be inserted into an expression vector so that the accessory function can be transferred to a cell. Alternatively, nucleic acid molecules comprising one or more accessory functions can be synthetically derived from Ad genomic sequence information and then inserted into cells by known means.

In one embodiment the recombinant adenovirus genome in the plasmids or cosmid of the invention is advantageously a complete genome. This is particularly useful since a second construct supplying another part of the viral genome and with the need for the recombination step in the encapsidating line may not be needed. In one embodiment, the plasmids or cosmids of the invention encompass a first region, which enables them to replicate in certain host cells, and a second region, which comprises the viral sequences or adenoviral sequences and expression cassette of the invention. The prokaryotic or eukaryotic origin of replication used can be any of a number known in the art, including conditional origins of replication that function only in the presence of added compounds or only in certain cells that possess trans-activating factors or sequences. For example, an origin of replication that is derived from a plasmid of incompatibility group P (pRK290), which allows replication in E. *coli* pol A strains, can be used. Other compatibility groups can be used in like manner. The viral or adenoviral sequences can conveniently be flanked by one or more restriction sites that are not present in the viral or adenoviral sequence for ease of further manipulation.

More preferably, the first region that allows replication in host cells may also encompass a region that enables the host cells containing the plasmid or cosmid to be selected. This region can consist, in particular, of any gene that confers resistance to a compound, in particular an antibiotic. Thus, genes that confer resistance to kanamycin (Kan^{r}), to ampicillin (Amp,^{r}), to tetracycline (Tet^{r}) or to spectinomycin, for example, are commonly used (Sambrook *et al., Molecular Cloning,* 1989 and 2001). Plasmids and cosmids can be selected using genes other than those genes that encode markers for resistance to an antibiotic. In general, a gene that confers on a host cell a function that the cell does not possess or no longer possesses can be used in this manner (this function can correspond to a gene which has been deleted from the chromosome or rendered inactive), with the gene on the plasmid establishing or providing this function. As an additional example, the gene can be a gene for a transfer RNA, which re-establishes a deficient chromosomal function. As used in this invention, the bioactive polypeptides or proteins of encoded by the expression cassette of the invention generally do not contain these selectable marker genes or resistance genes. They may, however, employ reporter genes, such as green fluorescent protein, an alkaline phosphatase or alkaline phosphatase-activity possessing fragment, or β-gal.

As previously indicated, the adenoviral genome present in the plasmids or cosmids of the invention advantageously contains a complete or functional genome, a genome that does not require other regions to be supplied by recombination or ligation in order to produce viral stocks in the chosen encapsidating lines. Preferably, however, the plasmids or cosmids contain only fragments or regions or a viral or adenoviral genome, encompassing certain functions, like the ITR sequences and/or a sequence that permits encapsidation.

The inverted repeat (ITR) sequences constitute the origin of replication of the adenoviruses. They are located at the ends of the viral genome, from where they can be readily isolated using standard molecular biological techniques that are known to the person skilled in the art. The nucleotide sequence of the ITR sequences of human adenoviruses (in particular serotypes Ad2 and Ad5) is described in the literature, as is that of canine adenoviruses (in particular CAV1 and CAV2). In Ad5, for example, the left-hand ITR sequence corresponds to the region encompassing nucleotides 1 to about 103 of the genome.

The encapsidation sequence (also termed Psi sequence) is required for encapsidating the viral genome. In the genome of wild-type adenoviruses, it is located between the left-hand ITR and the E1 region. It can be isolated or synthesized artificially using standard molecular biological techniques. The nucleotide sequence of the encapsidation sequence of human adenoviruses (in particular serotypes Ad2 and Ad5) is described in the literature, as is that of canine adenoviruses (in particular CAV1 and CAV2). In Ad5, a functional encapsidation sequence is contained between nucleotides 194 and 358 of the genome.

*Retrovirus vectors*. In another embodiment the nucleic acids, cassettes, and vectors and methods of the invention can be introduced or used in a retroviral vector, *e.g.,* as described in Parvean *et al., Nat. Biotech.* 18:623-629 (2000), Kim *et al., J. of Virol.* 72:994-1004 (1998); Salesse *et al., J. of Interfer. Cytokin. Res*., 20:577-587 (2000); US 5,399,346; Mann et al., Cell 33:153 (1983); US 4,650,764; US 4,980,289; Markowitz *et al., J. Virol.* 62:1120 (1988); US 5,124,263; EP 453 242; EP 178 220; Bernstein *et al., Genet. Eng.* 7:235 (1985); McCormick, *BioTechnology* 3:689 (1985); WO 95/07358; Kuo *et al., Blood* 82:845 (1993); Morgan *et al., Nucl. Acid. Res.,* 20:1293-9 (1992); and He *at al.,* Gene 175:121-125 (1996). The retroviruses are integrating viruses that infect dividing cells. The retrovirus genome includes two LTRs, an encapsidation sequence and three coding regions (gag, pol and env). In recombinant retroviral vectors, the *gag, pol* and *env* genes are generally deleted, in whole or in part, and replaced with a heterologous nucleic acid sequence of interest. These vectors can be constructed from different types of retrovirus, such as MoMuLV ("murine Moloney leukaemia virus"), MSV ("murine Moloney sarcoma virus"), HaSV ("Harvey sarcoma virus"), SNV ("spleen necrosis virus"), RSV ("Rous sarcoma virus"), and Friend virus. Some defective retroviral vectors are disclosed in W0 95/02697, for example.

In general, in order to construct recombinant retroviruses containing a nucleic acid or expression cassette of the invention, a plasmid is constructed that contains the LTRs, the encapsidation sequence, and a nucleic acid or expression cassette. This construct is used to transfect a packaging cell line, which is able to supply in *trans* the retroviral functions deficient in the plasmid. In general, the packaging cell lines are able to express the missing gag, pol and env genes. Packaging cell lines have been described and are known in the prior art, in particular the cell line PA3 17 (U.S. 4,861,719), the PsiCRIP cell line (WO 90/02806), and the GP+envAm12 cell line (WO 89/07150).

Packaging cell lines can also be derived from 293 cells by introducing the appropriate retroviral sequences. Preferably, retroviral vector sequences and structural gene sequences can be designed to minimize recombination to form replication-competent retrovirus. By introducing the gag-pol and env gene sequences into the packaging cell separately so that they integrate in different areas of the packaging cell genome, the rate of replication competent virus formation is decreased since multiple recombination events are required to generate replication-competent virus. The packaging function can also be inserted by chimeric vector systems, employing adenovirus vectors to introduce gag-pol and another adenovirus to introduce the expression cassette and the retroviral env. *See* Torrent *et al., Cancer Gene Ther.* 7:1135-44 (2000).

Retroviral vectors can be constructed to function as infectious particles or to undergo a single round of transfection. In the former case, the virus is modified to retain all of its genes except for those responsible for oncogenic transformation properties, and to express the heterologous gene. Non-infectious viral vectors are prepared to destroy the viral packaging signal. Thus, the viral particles produced are not capable of producing additional virus.

Plasmids containing retroviral genomes also are widely available from the American Type Culture Collection (ATCC), and other sources (*see, for example,* Gacesa and Ramji, *Vectors: Essential Data,* John Wiley & Sons, New York (1994)). The nucleic acid sequences of a large number of these viruses are known and are generally available from databases such as Genbank, for example. The complete nucleic acid sequence of the MoMLV and other MLVs is known in the art.

In one embodiment, isolated retroviral nucleic acid coding for the minimal gag-pol and env ORF is selected for use. In a preferred embodiment, the nucleic acid is selected from MLV and the minimal sequences used are, for example, nucleotides from about 621 to 5837 (gag-pol) (numbering from Shinnick et al. (1981)) and about nucleotides 37 to 2000 (env) (numbering from Ott et al. (1990)). The exact nucleotide positions will vary with different MLVs. Altered but functionally homologous or equivalent nucleic acid molecules can be selected and prepared by one skilled in the art. The minimal gag-pol and env ORF nucleic acid molecules can be isolated and published sequence information. For example, the sequence can be replicated by PCR, which, in combination with the synthesis of oligonucleotides, allows easy reproduction of DNA sequences.

*Additional Vectors, Plasmids, and Cosmids.* The invention also relates to plasmids and cosmids. These plasmids and cosmids may encompass a recombinant adenovirus genome, another viral genome, adenoviral shuttle vectors, or plasmids or cosmids bearing the cassettes and nucleic acids of the invention. As already described, the plasmids and cosmids can be used in generating the recombinant viruses using techniques known in the art. In addition, naked plasmids or cosmids can be used in a number of gene transfer protocols and the plasmids and cosmids of the invention can be used in this manner also. *See, in general,* Miyake *et al., PNAS* 93:1320-1324 (1996); US 6,143,530; US 6,153,597; Ding *et al., Cancer Res.,* 61:526-31 (2001); and Crouzet *et al*., *PNAS* 94:1414-1419 (1997). Plasmids can also be combined with lipid compositions, pharmaceutically acceptable vehicles, and used with electro-transfer technology, as known in the art. *See, for example,* U.S. Patents 6,156,338 and 6,143,729, and Bettan *et al*., Mol. Ther. 2: 204-210 (2000)).

Naked DNA has also been used as a gene transfer protocol. For example, numerous reports have discussed the use of naked DNA gene transfer for inducing angiogenesis, among others. The use in humans with an adaptation of balloon angioplasty to deliver the vector as well as intramuscular injections and direct application to tissue has been documented or discussed. *See, for example,* Isner, *Adv. Drug. Deliv. Rev.* 30:185-197 (1998); Tsurumi *et al., Circulation* 96(Suppl. 9):II-328-8 (1997); Takeshita *et al., Lab. Invest.* 75:487-501 (1996); Lewis *et al., Cardiovasc. Res.* 35:490-7 (1997).

### Exemplary Promoter and Promoter/Enhancer Regulatory or Control and Control Regions

As noted above, any appropriate promoter or promoter/enhancer can be selected and used to be linked to nucleic acid encoding the two or more bioactive polypeptides or proteins and drive expression of the two or more bioactive polypeptides or proteins in the nucleic acids and expression cassettes of the invention. As promoter and enhancer functions can be combined on a single nucleic acid, reference to promoter here can also mean a promoter combined with an enhancer, as known in the art. The description that follows merely lists and discusses preferred embodiments. In general, however, the promoter is functional in the host or target cell where expression is desired.

Preferred promoters include, but are not limited to, the cytomegalovirus immediate early promoter (CMV) (WO 96/01313; US 5,168,062; US 5,385,839), the human elongation factor 1α promoter (hEF1α), the SV40 early promoter region (SV40) (Benoist and Chambon, *Nature* 290:304-310(1981)), the promoter contained in the 3' long terminal repeat of Rous Sarcoma Virus (RSV) (Yamamoto, *et al., Cell* 22:787-797(1980)), the herpes simplex virus thymidine kinase promoter (HSV-TK) (Wagner *et al., Proc. Nat. Acad. Sci.* U.S.A. 78:1441-1445(1981)), and the regulatory sequences of the metallothionein gene (MT) (Brinster *et al., Nature* 296:39-42(1982)).

Any promoter or promoter/enhancer can be selected for use in the invention. Generally, however, promoters of similar expression level capabilities are selected to take advantage of the aspect of the invention that permits relatively equal levels of transgene expression from two or more polypeptide-encoding sequences. In a preferred embodiment in adenoviral vectors, two promoters are selected that possess similar activity in the particular cell(s) contemplated but which do not cause recombination between viral genomes.

An additional type of promoter that can be used is a tissue-specific promoter, of which many are known. For example, elastase I gene control region is active in pancreatic cells (Swift *et al., Cell* 38:639-646 (1984); Omitz *et al., Cold Spring Harbor Symp. Quant. Biol.* 50:399-409 (1986); MacDonald, *Hepatology* 7:425-515(1987)); insulin gene control region is active in pancreatic beta cells (Hanahan, *Nature* 315:115-122(1985)), immunoglobulin gene control region is active in lymphoid cells (Grosschedl et al., 1984, Cell 38:647-658 (1984); Adames *et al., Nature* 318:533-538 (1985); Alexander *et al., Mol. Cell. Biol.* 7:1436-1444 (1987)), mouse mammary tumor virus control region is active in testicular, breast, lymphoid, and mast cells (Leder *et al., Cell* 45:485-495 (1986)), albumin gene control region is active in liver (Pinkert *et al., Genes and Devel.* 1:268-276 (1987)), alpha-fetoprotein gene control region is active in liver (Krumlaufet al., Mol. Cell. Biol. 5:1639-1648 (1985); Hammer et al., Science 235:53-58 (1987)), alpha 1-antitrypsin gene control region is active in the liver (Kelsey *et al., Genes and Devel.* 1:161-171 (1987)), beta-globin gene control region is active in myeloid cells (Mogram *et al., Nature* 315:338-340 (1985); Kollias *et al., Cell* 46:89-94 (1986)), myelin basic protein gene control region is active in oligodendrocyte cells in the brain (Readhead *et al., Cell* 48:703-712 (1987)), myosin light chain-2 gene control region is active in skeletal muscle (Sani, *Nature* 314:283-286 (1985)), and gonadotropic releasing hormone gene control region is active in the hypothalamus (Mason *et al., Science* 234:1372-1378 (1986)).

In general, promoters that may be used in the present invention include both constitutive promoters and regulated (inducible) promoters. Tetracycline-regulated transcriptional modulators and CMV promoters are described in WO 96/01313, US 5,168,062 and 5,385,839, for example. An inducible promoter is a promoter that is inactive or that exhibits low activity except in the presence of an inducing substance. Some additional examples of inducible promoters include, but are not limited to, those identified as or associated with genes of MT II, MMTV, Collagenase, Stromelysin, SV40, Murine MX gene, α-2-Macroglobulin, MHC class I gene h-2kb, HSP70, Proliferin, Tumor Necrosis Factor, or Thyroid Stimulating Hormone alpha genes.

In addition, the promoter may be modified by addition of activating or regulatory sequences or sequences allowing a tissue-specific or predominant expression (enolase and GFAP promoters and the like). Moreover, when the nucleic acid does not contain promoter sequences, it may be inserted, such as into the virus genome downstream of such a sequence.

Hybrid or chimeric intron sequences can also be used in the expression cassettes and vectors. Huang and Gorman, Nucl. Acids Res. 18:937-47 (1990). A number of these sequences have been described in the art including those from rabbit beta-globin, CMV immediate early gene, IgG gene, human beta-globin gene first intron, the 5' UTR of the human elongation factor 1α gene, and individual donor and acceptor sites taken from any known intron. Other examples have been described above or are known in the art. Any functioning intron sequence can be selected for inclusion.

### Exemplary Uses of Gene Transfer Vectors, Plasmids, and Cassettes

Adenoviral expression vectors have become important tools in functional analysis of protein-encoding sequences. A number of available adenoviral-based systems have been developed for expression of genes in mammalian cells. The systems available from TaKaRa Biomedicals (Takara ShuzoCo., Ltd. Japan) and Qbiogene (Carlsbad, CA), for example, exhibit the use of adenoviral systems for convenient expression of genes and for the expression of toxic products. These systems, or systems like them, can also be used for large-scale production of recombinant proteins, especially those advantageously produced in mammalian or human cells. These systems can be adapted to express multiple protein-encoding sequences according to the invention, or elements of these systems can be adapted to produce the vectors, nucleic acids, recombinant viruses, or cassettes of this invention.

The vectors, recombinant adenoviruses, and nucleic acid cassettes of the invention can be used to express multiple genes in an adenoviral expression system. In an exemplary embodiment, two or more genes or nucleic acids can be expressed together to determine synergistic or complementary biological functions or effects. For example, a known cytokine can be expressed together with a mutant cytokine(s) or with different, novel nucleic acids (test sequence) identified from a screen of sequences implicating immune cell function, such as an expression profiling screen. In this way, the function of the mutant cytokine or the test sequence can be analyzed in a particular cellular environment. The cellular environment can be, for example, one that allows for the identification of synergistic functional relationships between the two or more expressed polypeptides.

In addition, for their use according to the present invention, the vectors, either in the form of a virus vector, nucleic acid-lipid composition, or naked DNA, are preferably combined with one or more acceptable carriers for an injectable formulation. These carriers can even be pharmaceutically acceptable carriers. The phrase "pharmaceutically acceptable" refers to compositions that are physiologically tolerable to animals and/or humans. The term "carrier" refers to a diluent, adjuvant, excipient, or vehicle with which the vector is administered. Examples of acceptable pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Saline, buffered saline, isotonic saline (e.g., monosodium or disodium phosphate, sodium, potassium, calcium or magnesium chloride, or mixtures of such salts), Ringer's solution, dextrose, water, sterile water, glycerol, ethanol can also be used. Suitable pharmaceutical carriers are also described in "Remington's Pharmaceutical Sciences" (Gennaro *et al*., eds.).

The virus or vector doses used for the administration may be adapted as a function of various parameters, and in particular as a function of the site of administration contemplated, the number of injections, the transgenes to be expressed, or the desired duration of treatment. In general, the recombinant adenoviruses according to the invention are formulated and administered in the form of doses of between 10² and 10¹⁴ iu, and preferably 10⁶ to 10¹¹ iu. The term iu (infectious unit) corresponds to the infectivity of a virus solution, and is determined by infecting an appropriate cell culture and measuring by FACS, generally after 1-2 days, the percentage of cells detected by an adenovirus-specific antibody. Techniques for determining the titer of a viral solution are well known in the art.

As shown in the Examples, the invention can be used in conjunction with the analysis of tumor growth or the treatment of tumors in animals, particularly solid tumors. Examples of solid tumors include sarcomas and carcinomas such as, but not limited to: fibrosarcoma, myxosarcoma, liposarcoma, chondrosarcoma, osteogenic sarcoma, chordoma, angiosarcoma, endotheliosarcoma, lymphangiosarcoma, lymphangioendotheliosarcoma, synovioma, mesothelioma, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, cystadenocarcinoma, medullary carcinoma, bronchogenic carcinoma, renal cell carcinoma, hepatoma, bile duct carcinoma, choriocarcinoma, seminoma, embryonal carcinoma, Wilm's tumor, cervical cancer, testicular tumor, lung carcinoma, small cell lung carcinoma, bladder carcinoma, epithelial carcinoma, glioma, astrocytoma, medulloblastoma, craniopharyngioma, ependymoma, pinealoma, hemangioblastoma, acoustic neuroma, oligodendroglioma, meningioma, melanoma, neuroblastoma, and retinoblastoma. The present invention also provides for treatment of or analysis of conditions known to proceed to or suspected of preceding to neoplasia or cancer, in particular, where non-neoplastic cell growth consisting of hyperplasia, metaplasia, or most particularly, dysplasia has occurred. The invention can also be directed to analysis or treatment of malignant or non-malignant tumors and other disorders involving inappropriate cell or tissue growth augmented by angiogenesis. Analysis and treatment of other hyperproliferative disorders is also specifically contemplated.

The preferred route of administration to a tumor is by direct injection into the tumor or other intratumoral administration. The tumor can be imaged using any of the techniques available in the art, such as magnetic resonance imaging or computer-assisted tomography, and the vector-containing composition administered by stereotactic injection, for example. Alternatively, if a tumor target is characterized by a particular antigen, a vector of the invention can be targeted to the antigen as known in the art, and administered accordingly. Although the methods of the invention are effective in inhibiting tumor growth *in vivo,* the vectors and methods of the present invention are advantageously used with other treatment modalities, including without limitation additional viral vectors, surgery, radiation, and chemotherapy. Chemotherapeutic agents such as, though not limited to, taxol, taxotere and other taxoids (US 4,857,653; 4,814,470; 4,924,011, 5,290,957; 5,292,921; 5,438,072; 5,587,493; EP 0 253 738; and W0 91/17976, W0 93/00928, W0 93/00929, and W0 96/01815), or other chemotherapeutics, such as cis-platin, etoposide and etoposide phosphate, bleomycin, mitomycin C, CCNU, doxorubicin, daunorubicin, idarubicin, ifosfarnide, and the like, are preferred. As noted and shown in the Examples, a vector of the invention can be administered in conjunction with another vector or gene transfer method, such as but not limited to those incorporating genes for delivering p53 or analogues thereof such as CTS- 1 (W0 97/04092), retinoblastoma (Rb), adenosine deaminase (Ada), mda-7 (Jiang *et al*., Oncogene 11:2477-2486 (1995)), thymidine kinase (TK) or analogues thereof, anti-RAS single chain antibodies, interferon-α, interferon-γ, tumor necrosis factor-α, tumor necrosis factor-β, interleukin-2, interleukin-7, interleukin-12, interleukin-15, interleukin-18, B7-1 T cell costimulatory molecule, B7-2 T cell costimulatory molecule, immune cell adhesion molecule (ICAM)-1 T cell costimulatory molecule, granulocyte colony stimulatory factor, granulocyte-macrophage colony stimulatory factor, and combinations thereof.

The vectors, nucleic acids, expression cassettes and methods of the invention can be used for the treatment of numerous other conditions and diseases of animals, including humans. The selection of transgenes and/or polypeptide-encoding sequences will determine the type of disease or condition the treatment is intended for.

Any vector can be used in conjunction with the present invention, such as a viral vector or naked DNA. In preferred embodiments, a single vector (virus or DNA) is used to deliver genes coding for both an anti-angiogenesis function and an anti-tumor function, or for a combination of two or more anti-angiogenesis proteins, or for a combination of two or more angiogenic proteins. Thus, in preferred embodiments the methods can be used for treating tumors, cancers, cardiovascular disease, stroke, heart attack, chronic heart disease, ischemic disease, and for diseases where re-vascularization improves or ameliorates tissue status or function.

### Exemplary Combinations of Protein or Polypeptide-Encoding Sequences for Multiple Expression from a Single Vector

The proteins or polypeptides that can be incorporated into the vectors, nucleic acids, cassettes, and recombinant viruses of the invention, and used in the methods of the invention, can be any combination of bioactive genes and/or sequences known or available. A "bioactive" gene or sequence encodes a protein or polypeptide that, when expressed, provides some action, function, effect, or structural product, the presence of which can be detected or indicated by any number of methods or assays. One skilled in the art is familiar with numerous means and methods for detecting the presence of recombinant proteins or polypeptides. The most straightforward are ELISA or FACS or other antibody-binding assays that indicate the presence of a protein or polypeptide. Numerous enzymatic, cell-based, or other functional assays also exist and can be used depending on the type of gene or sequence employed. *In vivo* assays can also be used, where the cell or an extract containing the gene or sequence or expressed protein or polypeptide has some biological or systemic effect on certain cells or tissue in an organism. The examples possible are numerous and will depend on the type of bioactive gene or sequence selected for use. The genes, sequences, polypeptides and proteins noted in this section are not an exclusive list of those capable of being used in the invention. They are discussed here only as preferred examples and not as a limitation to the scope of the embodiments.

Some of the exemplary categories of bioactive genes or sequences include, for example, those that encode the interleukin proteins, the interferon proteins, endostatin, angiostatin, thymidine kinases, the TNF proteins (tumor necrosis factor), and suicide proteins or apoptotic proteins. Other general categories include immunomodulatory genes, pro-drug activating enzyme genes, and genes involved in the cell growth or apoptotic processes of cells. Many particular examples have already been mentioned in this disclosure. In addition, viral, bacterial, animal, or even plant-derived proteins can be selected for use. Another category of protein or polypeptides is multi-chain or multi-subunit proteins that can be encoded by two or more separate sequences.

In addition, fusion polypeptides can be selected and used. A "fusion polypeptide" is a polypeptide comprising regions in a different position in the sequence than occurs in nature. The regions may normally exist in separate proteins and are brought together in the fusion polypeptide, or they may normally exist in the same protein but are placed in a new arrangement in the fusion polypeptide. A fusion polypeptide may be created, for example, by chemical synthesis, or by creating and translating a polynucleotide in which the peptide regions are encoded in the desired relationship.

Cell adhesion molecules, or CAMs, can be selected as aberrant expression of CAMs may be involved in the tumorigenesis of several neoplasms. For example, E-cadherin, α₅ β₁ integrin, and C-CAM. Tumor suppressors that may be employed include Rb, APC, DCC, NF-1, NF-2, WT-1, MEN-I, MEN-II, zac1, p73, BRCA1, VHL, FCC, MMAC1, MCC, p16, p21, p57, C-CAM, p27 and BRCA2. Inducers of apoptosis, such as Bax, Bak, Bcl-X, Bik, Bid, Harakiri, Ad E1B, Bad and ICE-CED3 proteases, and caspase proteins can also be used. Various enzyme genes can be used, including cytosine deaminase, hypoxanthine-guanine phosphoribosyltransferase, galactose-1-phosphate uridyltransferase, phenylalanine hydroxylase, glucocerbrosidase, sphingomyelinase, α-L-iduronidase, glucose-6-phosphate dehydrogenase, Akt serine/threonine kinases, HSV- thymidine kinase, and human thymidine kinase.

Peptide hormones are another group of genes that may be used in the vectors described herein. Included are growth hormone, prolactin, placental lactogen, luteinizing hormone, follicle-stimulating hormone, chorionic gonadotropin, thyroid-stimulating hormone, leptin, adrenocorticotropin (ACTH), angiotensin I and II, beta-endorphin, beta-melanocyte stimulating hormone (beta-MSH), cholecystokinin, endothelin I, galanin, gastric inhibitory peptide (GIP), glucagon, insulin, lipotropins, neurophysins, somatostatin, calcitonin, calcitonin gene related peptide (CGRP), beta-calcitonin gene related peptide, hypercalcemia of malignancy factor, parathyroid hormone-related protein (PTH-rP), parathyroid hormone-related protein (PTH-rP), glucagon-like peptide (GLP-1), pancreastatin, pancreatic peptide, peptide YY, PHM, secretin, vasoactive intestinal peptide (VIP), oxytocin, vasopressin (AVP), vasotocin, enkephalinamide, metorphinamide, alpha melanocyte stimulating hormone (alpha-MSH), atrial natriuretic factor (ANF), amylin, amyloid P component (SAP-1), corticotropin releasing hormone (CRH), growth hormone releasing factor (GHRH), luteinizing hormone-releasing hormone (LHRH), neuropeptide Y, substance K (neurokinin A), substance P and thyrotropin releasing hormone (TRH).

Another preferred class of genes contemplated include interleukins and cytokines, such as Interleukin 1 (IL-1), IL-2, IL-3, IL-4, IL-5, IL-6, IL-7, IL-8, IL-9, IL-10, IL-11 IL-12, IL-15, IL-18, GM-CSF, and G-CSF. Another preferred class of genes include antibodies or single-chain antibodies or fragments of antibodies.

Another preferred class of genes contemplated includes the group of proteins and polypeptides that promote vascular and endothelial cell growth and the numerous proteins and polypeptides derived from them. Examples include VEGF, VEGF₁₆₅, VEGF₁₂₁, VEGF₁₈₉, FGF, EG-VEGF, aFGF, aFGF₁₋₁₅₄, aFGF₂₄₋₁₅₄, bFGF, and FGF-5. These and other suitable proteins and/or polypeptides can be selected for therapeutic angiogenesis methods and treatments in conjunction with the invention. In addition, these factors, proteins, or polypeptide-encoding nucleic acid sequences of the endothelial and/or vascular growth or angiogenic activity can be mutated in *vitro* or *in vivo*, to create variations in coding sequence. Preferably, such mutations enhance the functional activity of the gene product. Any technique for mutagenesis known in the art can be used, including but not limited to, *in vitro* site-directed mutagenesis (Hutchinson, C., et al., J. Biol. Chem. 253:6551(1978); Zoller and Smith, DNA 3:479-488 (1984); Oliphant et al., Gene 44:177 (1986); Hutchinson et al., PNAS 83:710 (1986)). PCR techniques are preferred for site directed mutagenesis (see Higuchi, "Using PCR to Engineer DNA", in *PCR Technology: Principles and Applications for DNA Amplification*, H. Erlich, ed., Stockton Press, Chapter 6, pp.61-70 (1989)).

Additional examples of two-chain or multi-chain proteins that can be expressed in a single cell using the vectors and cassettes of the invention include enzymes such as malic dehydrogenase, glyceraldehyde-P dehydrogenase, aspartate carbamoyltransferase, α-keto acid dehydrogenases, enolase, phosphorylase kinase, cAMP-dependent protein kinase, ion channels and trans-membrane receptors such as AChR, NMDA receptor, AMPA receptor, kainate receptor, group I metabotropic glutamate receptors (G protein-coupled receptors), group II metabotropic glutamate receptors (G protein-coupled receptors), group III metabotropic glutamate receptors (G protein-coupled receptors), voltage-gated K+ channel, voltage-gated Ca+ channel, transport and motility proteins, such as tubulin (microtubules), myosin thick filament, tropomyosin, troponin, immune system proteins, such as immunoglobulins, complement components, B7-1, leucocyte integrins (LFA & CR3 & CR4), IL-2 R, IL-3 R, IL-4 R, IL-5 R, IL-6 R, IL-7 R, IL-12 R, IL-15 R, IL-18 R, GM-CSF R, IFN R, LIF receptor, PDGF R, TCR, MHC class I proteins, and other proteins such as hemoglobin, hemoglobin H, myoglobin, GTP-binding regulatory proteins (G proteins), and HDL R.

The nucleic acid or DNA sequence may also contain an antisense sequence, whose expression in a cell makes it possible to control the expression of certain genes, including genes related to promoting cell proliferation. This control may occur during transcription, splicing of the premessenger, degradation of the messenger, its translation into protein, or post-translational modifications. Preferably, the DNA sequence contains a gene encoding an antisense RNA capable of controlling the translation of a target mRNA (*see, for example,* EP 140 308). Among the antisense sequences that can be used in the invention or those sequences resulting in reduction in the levels of oncogenes ras, myc, fos, c-erb B, and the like.

Any combination of the proteins listed in this section can be selected for use with the invention.

### Exemplary Nucleic Acids and Genes Encoding Anti-Angiogenic Proteins

The vectors of the invention can be used to deliver a gene encoding an anti-angiogenic protein into a tumor in accordance with the invention. In a preferred embodiment, the anti-angiogenic factor is the amino terminal fragment of urokinase, containing the EGF-like domain. Such fragment corresponds to amino acid residues about 1 to about 135. In another embodiment, the anti-angiogenic factor maybe provided as a fusion protein, e.g., with immunoglobulin or human serum albumin as in, for example W0 93/15199, which is specifically incorporated herein by reference in its entirety.

Genes encoding other anti-angiogenic proteins can also be used according to the invention. Such genes include, but are not limited to, genes encoding angiostatin (O'Reilly *et al., Cell* 79:315-328 (1994); W0 95/29242; US 5,639,725), including angiostatin comprising kringles 1 to 3; tissue inhibition of metalloproteinase (*Johnson et al., J*. *Cell. Physiol.* 160:194-202(1994)); inhibitors of EGF or VEGF; and endostatins (W0 97/15666). In a preferred embodiment, the anti-angiogenic factor is angiostatin, particularly kringles 1 to 3 of angiostatin. In a particularly preferred embodiment, the anti-angiogenic factor is the amino-terminal fragment (ATF) of plasminogen having an amino acid sequence of plasminogen from about amino acid residue 1 to about residue 333 or a polypeptide comprising the kringle 1 domain of hATF. In another embodiment, the invention provides for administration of genes encoding soluble forms of receptors for angiogenic factors, including but not limited to soluble VGE/VEGF receptor, and soluble urokinase receptor (Wilhem *et al., FEBS Letters* 337:131-134(1994)).

In general, any gene encoding a protein or soluble receptor that antagonizes endothelial cell activation and migration, or which is involved in angiogenesis, can be employed in the vectors, nucleic acids, cassettes, and methods of the invention. The delivery of amino terminal fragments of plasminogen that function as angiostatin-like polypeptides are especially effective in this regard, for example. As noted above, modifications to protein sequence and protein or polypeptide analogues can be selected and used.

A gene encoding an anti-angiogenic factor, whether genomic DNA or cDNA, can be isolated from any source, particularly from a human cDNA or genomic library. Methods for obtaining such genes are well known in the art, as described above (*see, e.g.,* Sambrook et al., 1989). Due to the degeneracy of nucleotide coding sequences, other nucleic acid sequences which encode substantially the same amino acid sequence as an anti-angiogenic factor gene may be used in the practice of the present invention and these are contemplated as falling within the scope of the claimed invention. These include but are not limited to allelic genes, homologous genes from other species, and nucleotide sequences comprising all or portions of anti-angiogenic factor genes altered by the substitution of different codons that encode the same amino acid residue within the sequence, thus producing a silent change. Likewise, the anti-angiogenic factor derivatives of the invention include, but are not limited to, those containing, as a primary amino acid sequence, all or part of the amino acid sequence of an anti-angiogenic factor including altered sequences in which functionally equivalent amino acid residues are substituted for residues within the sequence resulting in a conservative amino acid substitution.

Additionally, a polypeptide, protein or more particularly the anti-angiogenic factor-encoding nucleic acid sequence can be mutated in *vitro* or *in vivo,* to create variations in coding sequence. Preferably, such mutations enhance the functional activity of the mutated anti-angiogenic factor gene product. Any technique for mutagenesis known in the art can be used, including but not limited to, *in vitro* site-directed mutagenesis (Hutchinson, C., et al., J. Biol. Chem. 253:6551(1978); Zoller and Smith, DNA 3:479-488 (1984); Oliphant et al., Gene 44:177 (1986); Hutchinson et al., PNAS 83:710 (1986)). PCR techniques are preferred for site directed mutagenesis (see Higuchi, "Using PCR to Engineer DNA", in *PCR Technologv: Principles and Applications for DNA Amplification*, H. Erlich, ed., Stockton Press, Chapter 6, pp.61-70 (1989)). In addition, homologs and ortholog sequences can be selected from databases by searching tblastx or other blast algorithms under the default setting for polypeptides with 95% amino acid identity, or about 90-95% identity, or about 80-95% identity, or about 50-75% or about 65-85% identity over a functional domain of the polypeptide or a region outside the functional domain.

### Exemplary Apoptotic, Toxic, or Suicide Nucleic Acids or Genes

One or more of the bioactive polypeptide-encoding or protein-encoding sequences used may comprise at least one gene chosen from a gene that is toxic to a cell, a suicide or apoptotic gene, a gene whose expression makes it possible to at least partially inhibit cell division or growth, a gene that encodes a protein that is capable of converting a compound into a toxic compound, or a gene encoding a lymphokine. Among the possible toxic and/or suicide genes preferred are those whose expression product confers on the cell a specific sensitivity to an agent that can be administered to the cell or organism. For example, the thymidine kinase gene, whose expression product confers on mammalian cells sensitivity to certain agents such as ganciclovir or acyclovir. The thymidine kinase of the herpes simplex virus is capable of phosphorylating nucleoside analogues, such as acyclovir or ganciclovir. The phosphorylated molecules, which can be subsequently further phosphorylated by cellular kinases, can be incorporated into a DNA chain undergoing elongation, which halts DNA synthesis, resulting in the death of the cell (*see, for example,* F.L. Moolten, Cancer Res. 46:5276 (1986)). Only the cells undergoing the synthesis of DNA or undergoing division, such as proliferating or tumor cells, are affected. The thymidine kinase gene of the human herpes virus (HSV-TK) is preferred. The sequence of this gene has been described in the literature (see especially McKnight *et al., Nucleic Acid Res.* 8:5931(1980)).

It is also possible to use the cytosine deaminase gene, whose expression product confers on mammalian cells sensitivity to 5-fluorocytosine (5-FC). Moreover, among the toxic genes which can be used within the framework of the present invention, there may also be mentioned the genes whose expression product induces apoptosis of the infected cell.

Among the genes whose expression makes it possible to at least partially inhibit cell division, tumor supressor genes (or anti-oncogenes) or any active derivative thereof can be used. Furthermore, antisense sequences- or ribozymes, whose expression in the target cell makes it possible to at least partially inhibit the expression of genes promoting cell division, can also be used. Among the tumor supressor genes that can be used within the framework of the present invention are the p53 gene (Baker et al., Science 244 (1989) 217); the Rb gene (Friend et al., Nature 323 (1986) 643; Huang et al., Science 242 (1988) 1563); the rap 1A gene (Kitayama et al., Cell 56 (1989) 77); the DCC gene (Fearon et al., Science 247: 49 (1990)), the k-rev2 and k-rev3 genes, or any other tumor suppressor genes described in the literature (*see, for example*, WO 91/15580).

Other embodiments include a "suicide gene" that allows recipient cells to be selectively eliminated at will. Isolation, insertion and use of such markers and suicide genes are well known to those of skill in the art as exemplified in WO 92/08796 and WO 94/28143. Numerous suicide genes are described in the literature, such as, for example, the genes coding for cytosine deaminase, purine nucleoside phosphorylase or a thymidine kinase such as, for example, the chickenpox virus or the herpes simplex virus type 1 thymidine kinases. Among these genes, the gene coding for herpes simplex virus type 1 thymidine kinase is most especially advantageous from a therapeutic standpoint since, in contrast to the other suicide genes, it generates an enzyme, thymidine kinase, capable of specifically eliminating dividing cells. This enzyme has a different substrate specificity from the cellular enzyme, and it has been shown to be the target of guanosine analogues such as acyclovir or ganciclovir (Moolten, *Cancer Res.* 46:5276 (1986)). The TK gene is also beneficial for a propagated toxicity effect ("bystander" effect). As shown in the Examples, this effect manifests itself in the destruction not only of the cells with incorporated TK gene, but also the neighboring cells. The mechanism of this process may be explained in three ways: i) the formation of apoptotic vesicles that contain thymidine kinase or phosphorylated ganciclovir, originating from dead cells, followed by phagocytosis of these vesicles by the neighboring cells, ii) transfer of the pro-drug metabolized by thymidine kinase, by a process of metabolic cooperation, from the cells containing the suicide gene to the cells not containing it, and/or iii) an immune response linked to regression of the tumor (Marini *et al., Gene Therapy* 2:655(1995)).

For a person skilled in the art, the use of the suicide gene coding for herpesvirus thymidine kinase is amply documented. In particular, the initial *in vivo* studies on rats having a glioma show regression of tumors when the HSV1-TK gene is expressed and when doses of 150 mg/kg of ganciclovir are injected (K. Culver *et al., Science* 256: 1550). The sequence of the gene coding for the herpes simplex virus type 1 thymidine kinase enzyme has been described in the *literature* (*see, in particular, McKnight Nucl. Acids Res.* 8:5949 (1980)). Natural variants of it exist, leading to proteins having a comparable enzyme activity with respect to thymidine, or ganciclovir (M. Michael *et al., Biochem. Biophys. Res. Comm.* 209: 966 (1995)).

A thymidine kinase variant, as in the variants discussed above for the anti-angiogenic factors, can also be used. In particular, variants with at least one mutation in the region corresponding to the ATP-binding site combined with at least one mutation in the N-terminal and/or C-terminal region. The mutation in the region corresponding to the ATP-binding site is preferably represented therein by at least one substitution of a guanine by an adenine at position 180 (G180A) *(see* WO 97/29196). The mutation in the N-terminal portion of the TK may be a substitution of the guanine by an adenine at position 16 (G16A) or a double substitution of the guanines at position 28 and 30 by adenines (G28A and G30A) and/or a double substitution of the cytosines at position 591 and 892 by thymines (C591T and C892T) and/or a double substitution of the guanines at position 1010 and 101 by adenines (G1010A and G1011A).

A number of therapeutic trials are also in progress in man, in which the TK gene is delivered to the cells by means of different vectors such as, in particular, retroviral or adenoviral vectors. In clinical trials of gene therapy in man, the doses which have to be administered are much smaller, of the order of 10 mg/kg per day or 5 mg/kg twice a day, and for a short treatment period (14 days) (E. Oldfield et al., 1995 Human Gene Therapy 6: 55). With higher doses or treatments over a longer period of time, adverse side effects are, in effect, observed. Similarly, other pro-drug converting enzymes can be used in place of the TK gene. Non-limiting examples include nitroreductase, carboxypeptidase G2, cytosine deaminase, lysosomal glucuronidase, human cytochrome P450, or variants of these or those discussed in gene-directed enzyme pro-drug therapy (GDEPT).

The following Examples are merely exemplary of the scope and content of this specification and should not be taken as a limitation of the invention to any specific embodiment. One of skill in the art is familiar with many techniques and teachings allowing the modification of these examples and the examples noted throughout this disclosure that would also employ the basic, novel, or advantageous characteristics of the invention. Thus, the scope of the invention is not limited by the particular examples listed here or elsewhere.

### Example 1: Construction of Adenoviral and Retroviral Vectors With End-to-End Cassette

As a preferred embodiment, adenoviral vectors can be used to produce vectors of the invention. Adenoviral vectors are preferred for numerous reasons, however, they are not the only type of vector contemplated or possible.

Adenoviral vectors can be used as convenient and flexible systems for testing the functional characteristics of protein or polypeptide-encoding sequences. For example, novel, uncharacterized sequences identified by expression profiling screening can be conveniently expressed in a number of different cells, including non-dividing cells, using adenoviral vectors. This strategy has been employed as a functional testing or analysis system. Since the expression profiling-derived sequences will often be selected because of a relationship to a known gene or group of genes, the present invention can be advantageously used to analyze the functional characteristics of a novel sequence in the context of being expressed in the same cell with a separate, known protein-encoding sequence.

In addition, adenoviral vectors can be used to test and analyze mutated, analog, derivatized, or modified protein-encoding sequences, or fragments of them, within the context of a single cell expressing known, cooperative proteins or polypeptides. For example, numerous proteins are known to function solely or primarily in the presence of one or more additional proteins. By using the vectors, nucleic acids, and cells of the invention, mutated or modified forms of a single protein can be tested with the wild type or natural form an additional protein or proteins. Often the additional protein or proteins provide cooperative functions that address a single pathway. Or, the additional proteins can address functions in a complementary pathway. In one example, two or more proteins can be expressed that affect apoptosis in a cell, but by different mechanisms. The adenoviral vectors of the invention can be used to analyze the function of a modified protein while the other, additional proteins are also expressed. This methodology can be used to identify important regulatory domains or regions in proteins.

In one embodiment, replication-competent adenoviral vectors can be used. E1A-deleted virus by a method using homologous recombination is well known in the art (McGory, *et al.*, Virology 163, 614-617(1988)). This method requires two plasmids, one a viral plasmid containing the entire Ad genome, and the other a transfer plasmid containing an E1A gene with the E1 A-mutant. The viral plasmid used may contain the entire Ad genome. The transfer plasmid used contains insertion sequences, such as sequences from the tetracycline gene of pBR322 (Jelsma *et al*., *Virology* 163: 494-502 (1988)). For recombination to produce adenovirus, the viral plasmid and the transfer plasmid are cotransfected into 293 cells by calcium phosphate mediated transfection. After 5 hours the precipitate was rinsed and the cells were overlayed with growth medium containing agarose to isolate viral plaques. At 7-10 days after the initial transfection viral plaques were isolated, plaque purified two times, and subsequently viral DNA was screened using restriction enzyme analysis and DNA sequencing. Viral stocks were purified by double cesium chloride gradients and quantitated by column chromatography as described in Huyghe, *et al., Human Gene Therapy* 6:1403-1416(1995)).

Similarly, replication-defective adenoviruses can be produced. Preferred vectors employ adenovirus type containing modifications to eliminate certain E3 functions (as described in Jones and Shenk, Cell 13:181-188 (1978); and Jones and Shenk Cell 17:683-689 (1979)), a mutant adenovirus that contains a deletion of the majority of the E1b dl55K gene that eliminates production of a functional Elbdl55K (McLorie, *et al. J. Gen. Virol.* 72:1467-1471(1991)), and the adenovirus vector ACNS3 (Wills, *et al. Human Gene Therapy* 5:1079-1088(1994)), in which the complete E1 region is replaced with an inserted expression cassette.

Conditional replicative adenoviruses can be employed also. Preferred examples are those utilizing an E1b mutation or deletion in the gene encoding the p55 polypeptide of adenovirus or those with mutations or deletions in the E1a p105, p107, or p130 polypeptides of Ad 5. These mutations or deletions specifically alter the ability of the viral polypeptide to bind to tumor suppressor proteins in the host cell. Specific examples can be selected from WO 00/24408 or U.S. 6,133,243, specifically incorporated herein for that purpose. *(See also* Hermiston, *J. Clin. Invest.,* 105:1169-1172 (2000).)

Retroviral vectors can also be used. The nucleic acid sequences of a large number of these viruses are known and are generally available from databases such as GenBank, for example. The complete nucleic acid sequences of the MoMLV and other MLVs, for example, are known in the art. Retroviral nucleic acids encoding the minimal gag-pol and env ORFs are inserted into the genomes of the packaging cell lines used to produce retroviral vectors. The nucleic acid can be selected from MLV and the minimal sequences used are, for example, nucleotides from about 621 to 5837 (gag-pol) (numbering from Shinnick et al. (1981)) and about nucleotides 37 to 2000 (env) (numbering from Ott et al. (1990)). The exact nucleotide positions will vary with different MLVs. Altered but functionally homologous or equivalent nucleic acid molecules can be selected and prepared by one skilled in the art. In general, in order to construct recombinant retroviruses containing a nucleic acid or expression cassette of the invention, a plasmid is constructed that contains the LTRs, the encapsidation sequence, 5 and a nucleic acid or expression cassette. This construct is used to transfect a packaging cell line (for example 293-derived cells containing the above-noted gag, pol, and/or env genes), which is able to supply in *trans* the retroviral functions deficient in the plasmid. The packaging cell lines are able to express the missing gag, pol and env genes so that the entire viral particles can be produced in the cell. Virus can be isolted from the packaging cell lines as described and known in the art.

### Example 2: Expression of Multiple Cytokines or Immunomodulatory Proteins

To evaluate the effects of multiple cytokines on single cells or the effect of cells producing multiple cytokines, the recombinant vectors, nucleic acids, and cells of the invention can be used. In this example, a recombinant adenovirus is constructed to contain various cassettes encoding cytokines, here GM-CSF and IL-2.

Three constructs containing the cytokines or immunomodulatory gene sequences were analyzed. 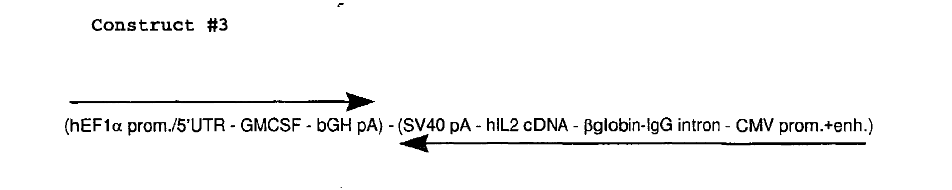
Construct #3


(hEF1α prom./5'UTR - GMCSF - bGH pA) - (SV40 pA - hIL2 cDNA - βglobin-IgG intron - CMV prom.+enh.)

 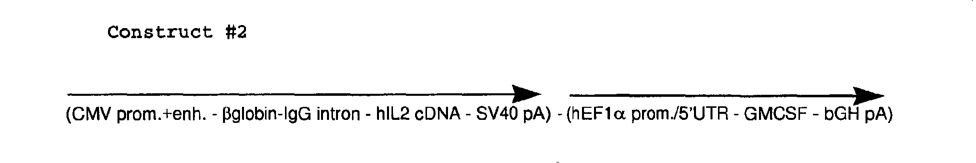
Construct #2


(CMV prom.+enh. - βglobin-IgG intron - hIL2 cDNA - SV40 pA) - (hEF1α prom./5'UTR - GMCSF - bGH pA)
 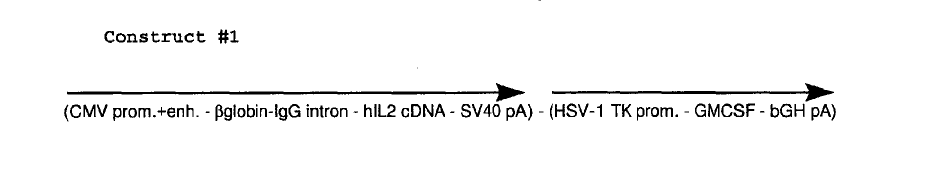
Construct #1


(CMV prom.+enh. - βglobin-IgG intron - hIL2 cDNA - SV40 pA) - (HSV-1 TK prom. - GMCSF - bGH pA)
 Each of constructs #1 and #2 contain the cytokines present in the same orientation relative to their reading frames (arrows above description of sequences indicate direction of reading frame). Construct #3, representing a cassette or nucleic acid of the invention, contains the cytokines oriented in opposite directions relative to their reading frames. These three constructs were prepared using standard recombinant DNA techniques. The GM-CSF encoding sequences can be taken from various publicly available sources, including GenBank, and European patent documents EP 337 359, EP 188 479, and EP 202 300 (each incorporated herein by reference). The IL-2 encoding sequences can be taken from various publicly available sources, including GenBank, U.S. Patents 4,738,927, 4,798,789, and 5,641,665, and Gray et al., Nature, 295:503 (1982).

Figure 5 depicts the levels of expression of GM-CSF and IL-2 from adenoviral vectors containing construct #3 in A549 human lung cancer cells *in vitro* determined by FACS. The recombinant vectors were prepared by homologous recombination using a plasmid containing the construct #3 DNA (*see, for example,* WO 96/5506 for a description of methods of preparing adenoviruses).

Adenoviral vectors containing a combination of prodrug converting enzymes and cytokine or immunomodulatory encoding sequences can also be prepared by as first-generation, replication-defective vectors based on the Ad5 backbone with deletions in the E1 and E3 regions, or later-developed vectors with other deletions as noted above *(see also* Qian *et al*., Circ. Res. 88: 911-917 (2001)). An adenoviral vector encoding HSV-TK gene can be used as the starting material. The vector expresses the HSV-TK gene under the control of the human cytomegalovirus immediate/early (CMV IE) promoter/enhancer. The adenoviral vector containing the cDNA for murine GM-CSF and human IL-2 can be constructed as follows. The sequences encoding each of the two cytokines were first constructed in separate plasmids. The mGM-CSF cDNA is under the control of the human elongation factor- 1 alpha (EF1α) promoter and 5'UTR (*see, for example*, U.S. Patent 5,266,491). It is followed by the bovine growth hormone polyadenylation signal. The region encoding hIL-2 consists of the human CMV IE enhancer and promoter, a chimeric human beta-globin/murine IgG intron, the hIL-2 cDNA, and the SV40 late polyadenylation signal. The expression cassette is produced for mGM-CSF and hIL-2 by isolating and cloning in an end-to-end orientation in the suicide shuttle plasmid pXL3474. The TK expression cassette was independently cloned into pXL3474. The pXL3474 plasmid contains the ITR and ψ regions of adenovirus 5 separated from the pIX region by a multiple cloning site which replaces the adenoviral E1 region. The final adenoviral backbone plasmids for the viruses are made by the technique of *E. coli*-derived RAd genomes (EDRAG), recombining the shuttle plasmids and pXL3215 (which contains the entire adenoviral genome with a lacZ expression cassette in the E1 deletion). The resulting plasmids are digested by PacI to liberate the adenoviral genomes, then transfected via Lipofectamine™ (Gibco-BRL) into 293 packaging cells. Three cycles of amplification yields the final viral stocks, which are purified by HPLC using Resource 15Q resin (Pharmacia, NJ).

### Example 3: Construction of pGM-CSF-IL-2 and Ad5-GM-CSF-IL-2

The transgene cassette for expressing cytokines such as GM-CSF and IL-2 can be produced from elements and sequences known in the art and from published sequences. Here, a EF promoter is linked to a GM-CSF sequence and the CMV promoter linked to an IL-2 sequence. The expression cassette is used in producing a plasmid and a recombinant adenovirus.

GM-CSF region. Human elongation factor-1α promoter and 5'UTR can be selected, as known in the art (*for example*, U.S. Patents 5,225,348, 5,266,491; Uetsuki, et al., J. Biol. Chemistry 264:5791-5798 (1989). The 5'UTR contains an intron, which itself contains a transcriptional enhancer. In the hEF1α gene, the initiating ATG is found 21 bp 3' of the end of the 5' UTR (intron) sequence used here. Also as used here, the ATG of hGM-CSF is 11 bp 3' of the end of the 5'UTR. Human, or in this case mouse granulocyte-macrophage colony stimulating factor cDNA, from a few bases 5' of the ATG initiating codon to the stop codon can be selected from numerous sources available (*see, for example,* EP 337 359, EP 188 479, EP 202 300, U.S. Patent 5,602,007). The ATG can preferably be modified to approach the Kozak consensus sequence, as known in the art. Bovine growth hormone poly A signal, from about 115 bp 5' of the AATAA site to about 50 bp 3' of the G/T-rich stretch, can also be selected (*see, for example,* Sasavage, N. L., et al., Biochemistry, 19:737-1743 (1980); Woychik, R. P., et al., PNAS, 81:3944-3948 (1984)). These sequences, or functional homologues or corresponding sequences, can also be selected from publicly available databases.

IL-2 region. Human cytomegalovirus enhancer and promoter can be selected from numerous sources (*see, for example* U.S. Patents 5,168,062, 5,385,839, and 5,641,665 and WO 96/01313). A hybrid β-globin/IgG intron containing the last 26 bp of the 1^{st} exon of the human β-globin gene, and the 1^{st} 104 bp of the 1^{st} hβ-globin intron, then the last 29 bp of the murine IgG intron (from the germ line V_{H} region (V6)), followed by the 1^{st} 19 bp of the following intron *(see* Bothwell *et al*., Cell 24:625 (1981)) can be used. Human interleukin-2 cDNA, from the ATG initiating codon to the stop codon can be selected from numerous sources (*see* U.S. Patents 4,738,927, 4,798,789, and 5,641,665, and Gray et al., Nature, 295:503 (1982), *for example*). The SV40 late poly A signal can be selected, from 120 bp 5' of the late AATAA to 50 bp 3' of the T-rich stretch. (*See, for example,* Reddy et al., DNA 6:461-72 (1987), Hsiung et al. App. Genet. 2:497 (1984), and U.S. Patent 4,798,789).

In one way of generating the cassettes, plasmids, nucleic acids, or recombinant viruses of the invention, each of the above mentioned sequences, or functional or homologous equivalents, exist on separate plasmids or a combination of plasmids. These plasmids are used as starting material, as described in the reference cited above. Conventional recombinant DNA manipulations, PCR techniques, and/or EDRAG recombination (Crouzet *et al.* PNAS 94:1414-1419 (1997)), allow one skilled in the art to produce cassettes or nucleic acids as depicted in the Figures 1, 2A, 2B, 3, 4A, 4B.

For example, a representative scheme is produced below. The plasmids referred to are exemplary and denote steps rather than a specific starting material, although one skilled in the art can select actual plasmids for the uses indicated from those described in the art. A plasmid cloning vector (Marsh *et al*., Gene 32(3):481-5 (1984), for example) can be used by first inserting a polylinker sequence from the numerous polylinker sequences available, creating pClon. An insert containing the CMV enhancer + promoter sequence is isolated from an available source (pCMV). A second insert containing the hIL-2 coding sequence desired + SV40 late poly A site is amplified by PCR from a plasmid containing these sequences (pIL-2), and isolated for insertion into the cloning vector. The three components (vector plus two inserts) are then digested with appropriate enzymes for combination into a single plasmid (pC-IL-2). To pC-IL-2, intron sequences from human beta-globin and IgG genes can be added (βg/IgG) generating pCIL2-In.

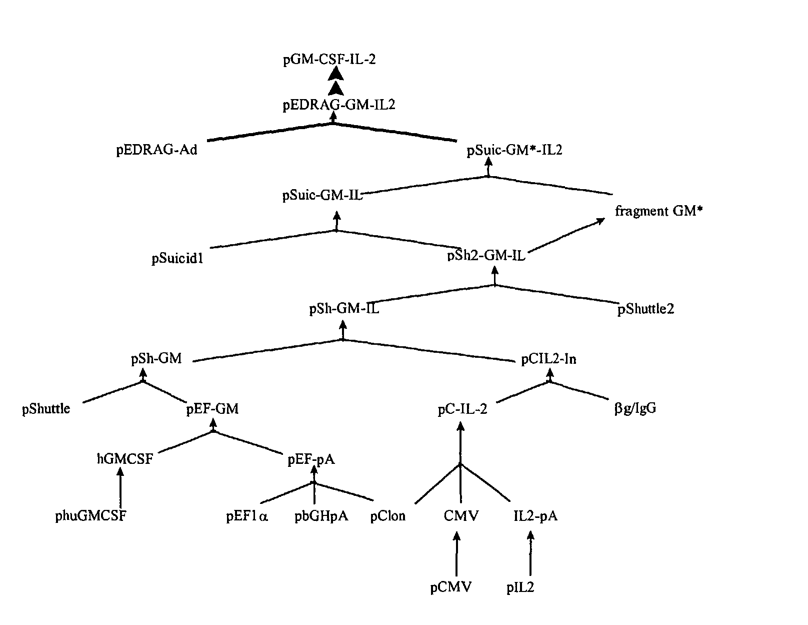

The cloning vector pClon can also be used to transfer a poly A site and promoter/enhancer site from separate plasmids (pEF1a, pbGHpA) into appropriate sites in the polylinker of the pClon to generate pEF-pA. This plasmid can be combined with a sequence coding for human GM-CSF to create pEF-GM. A shuttle vector (pShuttle) is used to move the promoter-coding sequence-poly A fragment from each of pEF-GM and pCIL2-In into one vector (pSh-GM-IL) so that the coding sequences are in opposite orientation with respect to their reading frames.

A second shuttle vector (pShuttle2) can be used to facilitate recombination with a suicide shuttle vector (pSuicid1) in preparing recombinant Ad vectors, as known in the art *(see, for example,* Crouzet *et al*., PNAS 94:1414-1419 (1997)). The suicide shuttle vector used can be any known in the art, including those designed for use in 293 cells or for use in PER.C6 cells. For example, a plasmid having Ad5 sequences of the ITR-ψ region, and Ad5 sequences from the pIX region, and in between a polylinker sequence to simplify insertion of other sequences. The Ad sequences allow for homologous recombination of the inserted sequence into an adenovirus. Various fragments of the pIX region or the entire pIX ORF can be used. As noted, many different variations in the suicide shuttle vector can be employed and the use of ITR-ψ and pIX regions is not required. The desired position of the insertion into the final recombinant adenovirus should be considered in selecting a suicide shuttle vector.

In the scheme above, a fragment containing the start of the mGM-CSF coding region that includes the ATG is removed from the pSH2-GM-IL in order to optimize the ATG environment to resemble the Kozak consensus sequence. PCR amplification using primers that incorporate the Kozak consensus sequence are used for this purpose. This results in higher expression levels in certain vector-cell combinations. Preferably, cassettes of the invention do incorporate a consensus Kozak sequence. The fragment containing the optimized ATG region (fragment GM*), which has been incorporated into the fragment by the PCR amplification, is reinserted into the suicide shuttle vector.

The EDRAG procedure can then be performed according to the method of Crouzet *et al*., noted above. First, plasmid pEDRAG-Ad containing the backbone of a selected Adenovirus, such as Ad5, is used in conjunction with the pSuic-GM*-IL2 plasmid in JM83 host, or the appropriate host (Yannisch-Perron, *et al*., Gene 33:103-119 (1985)). The resulting homologous recombination (noted in bold in the scheme above) generates plasmid pEDRAG-GM-IL2, where the GM-CSF and IL-2 cassette has been recombined with the Ad sequences on the pEDRAG plasmid. A second EDRAG selection, noted by the bold arrows in the scheme, is an intramolecular recombination designed to remove the extraneous plasmid sequences. This second homologous recombination can be performed according to the method of Crouzet *et al.* and in JM83 cells. The final proviral plasmid results (pGM-CSF-IL-2). The final proviral plasmid typically contains the sequence intended to be inserted into an adenovirus, here the GM-CSF-IL-2 expression cassette, flanked on both ends by the adenoviral sequences that define the insertion site in the adenoviral genome used.

In one example, depicted in Figure 13, the expression cassette is inserted into an E1 deletion site in Ad5. Accordingly, the ITR-ψ region of Ad5 and part of the pIX region that follows the E1 deletion (386-3512) is used in the suicide shuttle vector. In a first recombination step, the plasmid (pSuic-GM*-IL2) recombines via the pIX region with the homologous region in the adenoviral plasmid pEDRAG-Ad. pEDRAG-AD, here, carries the entire Ad5 genome, with an RSV LTR-LacZ cassette substituted for the adeno E1 region. This produces pEDRAG-GM-IL2. The second recombination step is intramolecular, between the two ITR-ψ regions, and leads to the elimination of the lacZ cassette, producing the final proviral plasmid, pGM-CSF-IL-2. The suicide shuttle vector is grown in the XAC-1 pir bacterial strain (Soubrier *et al*., Gene Therapy 6:1482-1488 (1999)). The recombination steps are carried out in the JM83 strain (Yannisch-Perron *et al.* (1985) Gene 33:103-119).

The proviral plasmid can be digested to linearize it and to isolate the recombinant adenoviral genome from the bacterial vector sequences. This material can be transfected into the PER.C6 packaging cell line. Fourteen days later enough virus is generated to produce a cytopathic effect, which can then be amplified several times to produce the final viral stock.

Various methods for maintaining and propagating adenovirus exist. The packaging cell lines, 293 and PER.C6 can be used. The cells are cultured in DMEM with 10% of fetal calf serum (FCS), or the equivalent. Viral infection is performed in 2% FCS, or 5-10% FCS, optionally substituted with 3 ng/ml of recombinant human b-FGF. The multiplicity of infection (MOI) can be calculated by known methods.

### Example 4: Construction of Angiogenic Factor-expressing Vectors and Nucleic Acids

A recombinant adenovirus that expresses a combination of anti-angiogenic factors can be produced as per the methods described above. Furthermore, plasmids for expressing the combination of factors can also be made as per above. Both naked plasmid techniques and recombinant virus techniques have been used to deliver anti-angiogenic factors to mammalian cells. In addition, certain anti-angiogenic factors have been used in the clinical arena. For example, endostatin, IFN-alpha, IFN-gamma, and IL-12 have all been used in humans to prevent angiogenesis associated with tumor formation.

Various fragments of plasminogen that comprise an angiostatin-like function, such as those comprising the kringle 1-3 domain of plasminogen, can be selected as one factor. Fragments of collagen XVIII comprising the NC1 domain, endostatin, can be used as a second. Sequences and variant sequences that code for these polypeptides or functionally equivalent polypeptides, as well as methods for testing their anti-angiogenic characteristics, are known in the art *(see, for example,* Yokoyama *et al*., Cancer Res. 60:2190-6 (2000); Soff, GA, Cancer Metastasis Rev. 19(1-2):97-107 (2000); Kassam, *et al*., J. Biol. Chem. Manuscript M009071200, Dec. 12, 2000; O'Reilly, *et al*., Cell 79:315-328 (1994); Regulier, E. Cancer Gene Therapy 8:45-54 (2001); Joki, *et al*., Nat. Biotechnol. 19:35-9 (2001); Sim, *et al*., Cancer Metastasis Rev. 19:181-90 (2000); Ding, *et al*., Cancer Res. 61:526-31 (2001)). In addition, precursors and strategies for delivering precursors to generate anti-angiogenic functions to a cell or tissue are also known and can be incorporated into the vectors, recombinant viruses, and nucleic acids of the invention *(see, for example,* Matsuda *et al*., Cancer Gene Ther. 7:589-96 (2000); Ferreras *et al*., FEBS Lett 486:247-51 (2000)).

In one embodiment, the coding region for a fragment of human plasminogen (up to residue 333) is linked to the CMV promoter and SV40 poly A site. The cDNA sequence can be obtained from databases and public sources, such as those noted above or elsewhere. Optionally, a fragment encoding a signal peptide can be substituted for the amino terminal region of the plasminogen sequence, about 18 amino acids. The additional sequence encoding the mature plasminogen (Plg) up to a site near amino acid 333 can then be used to insert an appropriate stop codon and for linking the poly A site. A synthetic oligodeoxynucleotide encoding residues from about 327 to 333, for example, can be added to the mature Plg sequence by PCR amplification of the fragment. The synthetic oligo also includes a restriction site for adding the SV40 late poly A sequence 3' to the coding sequence. Alternatively, for flexibility in inserting other sequences, a polylinker site can be added by the synthetic oligo amplification step. This entire insert can be combined with a second recombinant insert encoding a second anti-angiogenic factor, such as an endostatin, or a sequence encoding a polypeptide for which the biological effect in combination with angiostatin will be tested *in vivo* or *in vitro* (test polypeptide). A plasmid containing the second recombinant insert can be constructed in like manner as one skilled in the art knows. According to the invention, the reading frames of the two polypeptides are oriented in opposite directions. The result is a plasmid that contains a cassette as shown in Figures 1, 2A, and 2B. Here, NA #2 would be angiostatin polypeptide and NA#1 would be endostatin polypeptide or the test polypeptide. Of course, NA#2 could also be endostatin and NA#1 the test polypeptide. As noted above, the test polypeptide can be adapted from or identified from a genomic screening process, such as expression profiling or database searches. As above, the EF-1α promoter/enhancer and bGH poly A can be used for NA#1. The plasmid can be used itself to express the anti-angiogenic factor(s), or used in a method to produce a recombinant virus that expresses the factor(s).

For generation of a recombinant adenovirus, for example, the nucleic acid (e.g., an isolated DNA fragment, plasmid, cosmid) carrying the angiostatin or test polypeptide expression cassette is combined with a vector (e.g., restricted adenoviral DNA, plasmid, cosmid, YAC) encoding the adenoviral genome. Homologous recombination in packaging cells or bacterial cells (depending on the vector selected) then takes place, as known in the art. Alternatively, the expression cassette can be directly cloned into a plasmid containing the adenoviral genome. Briefly, the nucleic acid containing the two polypeptide-encoding sequences in opposite orientation is first modified to contain adenoviral sequences at each end of the expression cassette. These adenoviral sequences are selected based upon the intended insertion site in the adenoviral genome. As discussed above, another vector containing a desired adenoviral genome, such as used in the EDRAG technique, or as in many of the E1 deleted, E3 deleted, or E4 deleted genomes known in the art, is also transfected into the 293 cell (i.e. a plasmid containing the first 6.3 kb of the Ad5 genome with a deletion between position 382 to 3446 or about 3446 of the E1 region; or a plasmid as described or referred to herein). The expression cassette will then insert into the recombinant adenoviral genome at the sites homologous to those at the end of the expression cassette. Once in the packaging cell, the recombinant genome can then be packaged into virus particles using packaging cell function of the 293 cells, as known in the art. Viral stocks are prepared and titrated as known in the art. These infection conditions are compared to those using the same adenovirus with a β-gal insert, and results in 80 and 65% of β-galactosidase-expressing cells. Alternatively, the number of particles per cell can be calculated by titrating the number of viral particles through HPLC analysis or plaque-counting.

### Example 5: Expression of Bioactive Polypeptides

The pGM-CSF-IL-2 plasmid of Figure 12, prepared as in the method above, is used to prepare recombinant adenoviral stock for infecting mammalian cells and cell lines. A replication-defective Ad5 virus with a E3 deletion can be selected, however many other different adenovirus backbones, including replication-competent adenovirus, can also be selected. A map of the recombinant adenovirus to be used is shown in Figure 13.

Human and other mammalian cells can be used for the expression. In Figures 5 and 6, A549 human lung cancer cells and 4T1 murine mammary cells are selected, respectively. The cells are cultured as known in the art and virus stock added to the indicated viral particle per cell (MOI). After two days, the expression levels of GM-CSF and IL-2 are determined for recombinant adenoviruses containing the nucleic acid expression cassettes of the invention (samples labeled #3) in comparison to adenoviruses where the two nucleic acids encoding the polypeptides are positioned in the same orientation with respect to their reading frames (#1 and #2).

FACS is a routine method for determining levels of protein expressed by cells by calculating the number of cells in a population that possess the protein *(see* Ausubel *et al*., Current Protocols in Molecular Biology). In Figure 5, FACS results show very efficient infection and expression levels in A549 cells. In each sample where virus is added, expression of both IL-2 and GM-CSF can be detected. The lack of IL-2 #2 positive cells is because the IL-2 expression level in those cells is below the detection level used in these results. Increases in expression levels with increased MOI is as expected. The highest levels of expression occurs with the recombinant adenovirus of the invention, #3. The standard error in the IL-2 sample #1 is quite high in comparison to sample #3, indicating a higher and more reliably consistent expression level for the virus of the invention.

Figures 6A and 6B show results of FACS comparing the number of expressing cells using a recombinant adenovirus of the invention (AV-GM/IL2 #3) to an empty adenovirus control (AV-empty) in 4T1 mammary cells. With MOI 1000 and above, the percentage of cells expressing GM-CSF and IL-2 compared to control is clearly detectable.

Figures 6C and 6D show protein levels measured by standard ELISA techniques known in the art. The amount of GM-CSF and IL-2 produced per 10⁶ cells in a 24 hr period is relatively equal, approximately 37 ng/ml of GM-CSF to approximately 48 ng/ml of IL-2. This represents merely a 30% difference between the two separate polypeptides, here expressed from nucleic acids linked to different promoters. The relative equality of the actual protein levels (gray bars) is very advantageous. Without being bound by any particular theory, we believe these results reflect the use of the cassette containing reading frames oriented in opposite directions. No protein is detected in the control samples in panels 6C and 6D (striped bars not present).

### Example 6: Activity of Expressed Polypeptides In Vivo

A recombinant adenovirus system for expressing HSV-TK (thymidine kinase) has been previously described and used (WO 97/29196; US 5,631,236; US 5,601,818, for example) and is a well characterized approach to analyzing tumor cell growth and cancer treatments. An adenoviral vector for expressing human HSV-TK is used in conjunction with an adenoviral vector of the invention containing the IL-2 and GM-CSF coding regions, as noted above. Figure 7 depicts the results of the treatment with the two adenoviral vectors over a period of 40 days, in combination with ganciclovir treatment. The tumor volume of the implanted 4T1 tumor cells shows a remarkable reduction in only the treatment employing the vector of the invention (AV-TK+AV-GM/IL2 #3). The comparative examples using the constructs # 2 and #1, where the same GM-CSF and IL-2 coding regions are used but in the same orientation, do not exhibit the same levels of reduction. Clearly, the vectors and methods of using the vectors of the invention are surprisingly superior to other vectors and methods.

The results are even more striking in Figure 8. In panel A, where Line01 cells have been introduced into the mice, the combination of the adenovirus of the invention and the TK adenovirus plus systemic ganciclovir administration results in an almost complete regression of tumor growth *in vivo.* In panel B, the same treatment regimen was used for mice that had 4T1 tumors cells introduced. Again, the combination of the expression cassette of the invention (dark circles) shows the best tumor reduction levels.

In Figure 9, the same experiment is performed and analyzed for the ability of mice to survive 4T 1 tumor cell implantation. Only in the treatment that utilizes the expression cassette of the invention is there any success in preventing death.

The above results clearly show the benefit of vectors comprising the expression cassette of the invention over comparative examples and controls.

Figure 10 displays the protein levels expressed from from tumors injected intratumorally with the GM-CSF/IL-2 adenovirus described in this invention. Tumors of 40 to 60 mm³ were injected intratumorally with 5x10¹⁰ VP of AV-GM/IL2. Fourty-eight hours later, a single tumor cell suspension was prepared. Tumor cells from control injected and AV-GM/IL2 injected tumors were cultured in complete medium for 24 hours and secreted mGM-CSF and hIL-2 levels were determined by ELISA. Secreted cytokine levels are presented as pg/ml for 1x10⁶ cells per 24 hours. AV-empty injected tumors did not demonstrate any significant secretion of mGM-CSF or hIL-2. However, tumor cells isolated from AV-GM/IL2-injected tumors demonstrated a significant cytokine secretion. Although some mouse to mouse variations in cytokine secretion are observed, both cytokines, mGM-CSF and hIL-2, are expressed at equal levels when tumor cells are derived from the same tumor-bearing mouse. This directly supports the claim that the expression cassette of the invention leads to high and equal expression *in vitro* as well as *in vivo.*

Figure 11 shows a plasmid map incorporating the expression cassette (i.e. IL-2 and GM-CSF) in these experiments. For each of the *in vivo* experiments in Figures 7-10, the adenovirus vectors are administered by intratumoral injection using conventional pharmaceutically-acceptable buffer or composition.

### Example 7: Plasmids Comprising Angiogenic Factors

Plasmids for use in gene transfer can be constructed with the expression cassettes or nucleic acids of the invention to deliver multiple angiogenesis-promoting transgenes. A non-limiting example is a plasmid for expressing a VEGF polypeptide and a FGF polypeptide.

A cDNA encoding a bioactive VEGF polypeptide, such as the 165 fragment of hVEGF (VEGF(165)), is inserted into a eukaryotic expression vector downstream of a promoter sequence, such as the CMV promoter/enhancer. The eukaryotic expression plasmid used can be one of many available and suitable for this purpose, include the pUC vectors, such as pUC118. These vectors will also contain convenient origins of replication in *E. coli* and an SV40 origin of replication, and a selection gene, such as β-lactamase. Downstream and in the opposite orientation from the reading frame of the VEGF gene, a second fragment comprising a FGF gene is inserted into the plasmid. This fragment also contains a eukaryotic promoter/enhancer sequence 5' to the FGF coding region, such as the EF 1 α promoter. For example, the native bFGF sequence can be used with the addition of an appropriate secretory signal, as known in the art, or an aFGF fragment, aFGF(1-154) or aFGF(27-154), can be used.

The plasmid can then be transfected into and tested in 293 cells to assess expression levels of the polypeptides selected. As in the adenoviral examples above, intron sequence and consensus sequences can also be used to optimize expression levels. The administration method preferred for these plasmids is intramuscular injection. Experimental models include the rabbit hindlimb ischemia model (*see* Tsurumi *et al*., Circulation 94:3281-3290 (1996)). However, other administration techniques can be used.

## Claims

**1.** An expression cassette comprising a nucleic acid molecule, wherein the nucleic acid molecule comprises two transgenes operably linked to selected regulatory regions so that the expression cassette comprises, from 5' to 3', a transcriptional promoter, an intron sequence, a first transgene, a poly A site, a poly A site, a second transgene, an intron sequence, and a transcriptional promoter, wherein the two transgenes are oriented in opposite direction with respect their reading frames or transcription start and stop sites, and wherein the expression cassette is capable of expressing the transgenes in a mammalian cell.

**2.** The expression cassette of claim 1, wherein one of the transgenes is a polypeptide-encoding sequence that encodes a polypeptide selected from the group consisting of: IL-2, α-interferon, γ-interferon, G-CSF, GM-CSF, and tumor necrosis factor alpha (TNFα).

**3.** The expression cassette of claim 1, wherein one of the transgenes is a polypeptide-encoding sequence that encodes a polypeptide selected from the group consisting of: angiostatin, endostatin, an amino-terminal fragment of plasminogen having an amino acid sequence of plasminogen from about amino acid residue 1 to about residue 333, FGF, aFGF, bFGF, VEGF, or an angiogenic fragment of FGF, aFGF, bFGF, VEGF.

**4.** The expression cassette of claim 1, wherein one of the transgenes is a polypeptide-encoding sequence that encodes a thymidine kinase, an Akt protein kinase, or a cytosine deaminase.

**5.** The expression cassette of claim 1, wherein one of the transgenes is a polypeptide-encoding sequence that encodes a polypeptide selected from the group consisting of: p53, Rb, mda-7, rap 1A, DCC, k-rev2, k-rev3, and adenosine deaminase.

**6.** A mammalian cell comprising the expression cassette of one of claims 1-5, or progeny of the cell.

**7.** A method of introducing a vector into a mammalian cell, comprising providing a vector containing the expression cassette of one of claims 1-5, and allowing the vector to contact the cell under conditions that permit the vector to enter the cell.

**8.** A cell produced from the method of claim 7.

**9.** A mammal containing a cell produced from the method of claim 7.

**10.** The expression cassette of claim 1, wherein a transcription promoter is the immediate early gene promoter of CMV.

**11.** The expression cassette of claim 1, wherein a transcription promoter is the promoter of the human elongation factor-1α gene.

**12.** The expression cassette of claim 1, wherein a poly A site is the SV40 late poly A site.

**13.** The expression cassette of claim 1, wherein a poly A site is the bovine growth hormone poly A site.

**14.** A plasmid comprising an expression cassette of one of claims 1-5 or 10-13.

**15.** An adeno-associated virus vector comprising an expression cassette of one of claims 1-5 or 10-13.

**16.** An adenovirus vector comprising an expression cassette of one of claims 1-5 or 10-13.

**17.** A cell comprising a plasmid or vector of one of claims 10-13.

**18.** An animal comprising a plasmid or vector of one of claims 10-13.

**19.** A method of inhibiting the growth or proliferation of a tumor in a mammal comprising introducing a vector comprising an expression cassette of claim 1 into a cell of the mammal by intratumoral administration, wherein one of the first or second transgenes encodes a cytokine or immunomodulatory polypeptide.

**20.** A method of inhibiting the growth or proliferation of a tumor in a mammal comprising introducing a vector comprising an expression cassette of claim 1 into a cell of the mammal by intratumoral administration, wherein one of the first or second transgenes comprises a suicide gene or a pro-drug converting enzyme gene.

**21.** The method of claim 19, wherein one of the first or second transgenes encodes IL-2.

**22.** The method of claim 19, wherein one of the first or second transgenes encodes GM-CSF.

**23.** The method of claim 20, wherein one of the first or second transgenes encodes thymidine kinase.

**24.** The method of claim 23, wherein one of the first or second transgenes encodes GM-CSF.

**25.** The method of claim 19, wherein one of the first or second transgenes encodes an Akt kinase.

**26.** A method of detecting a bioactive polypeptide comprising providing an expression cassette consisting of a nucleic acid molecule, wherein the nucleic acid molecule comprises a bioactive polypeptide-encoding sequence and a test polypeptide-encoding sequence, each sequence operably linked to selected transcriptional regulatory regions, wherein the two polypeptide-encoding sequences are oriented in opposite direction with respect their reading frames, administering a vector containing the expression cassette into a cell, expressing the two polypeptides in the cell, and detecting a change in a specific stimulatory response, biological property, or characteristic of the cell.

**27.** A method of detecting a bioactive polypeptide comprising providing an expression cassette consisting of a nucleic acid molecule, wherein the nucleic acid molecule comprises a first bioactive polypeptide-encoding sequence selected from sequences encoding an anti-angiogenic polypeptide and a second test polypeptide-encoding sequence, each sequence operably linked to selected transcriptional regulatory regions, wherein the two polypeptide-encoding sequences are oriented in opposite direction with respect their reading frames, administering the expression cassette into a cell, expressing the two polypeptides in the cell, and detecting a change in a specific stimulatory response, biological property, or characteristic of the cell.

**28.** The method of claim 27, wherein the cell is an endothelial cell.

**29.** The method of claim 27, wherein the first bioactive polypeptide is angiostatin or endostatin.

**30.** A method of detecting a bioactive polypeptide comprising providing an expression cassette consisting of a nucleic acid molecule, wherein the nucleic acid molecule comprises a first bioactive polypeptide-encoding sequence selected from sequences encoding an angiogenic polypeptide and a second test polypeptide-encoding sequence, each sequence operably linked to selected transcriptional regulatory regions, wherein the two polypeptide-encoding sequences are oriented in opposite direction with respect their reading frames, administering the expression cassette to a cell, expressing the two polypeptides in the cell, and detecting a change in a specific stimulatory response, biological property, or characteristic of the cell.

**31.** A method of treating ischemic tissue damage in an animal comprising introducing a vector comprising an expression cassette of claim 1 into an animal, wherein the first or second transgene is a polypeptide-encoding sequence encoding an angiogenic polypeptide, and causing the angiogenic polypeptide to be expressed by a cell of the animal.

**32.** A method of treating cardiovascular disease in an animal comprising introducing a vector comprising an expression cassette of claim 1 into an animal, wherein the first or second is a polypeptide-encoding sequence encoding an angiogenic polypeptide, and causing the angiogenic polypeptide to be expressed by a cell of the animal.

**33.** The method of one of claims 31-32, wherein the angiogenic polypeptide is selected from the group consisting essentially of : FGF, aFGF, bFGF, VEGF, or an angiogenic fragment of FGF, aFGF, bFGF, VEGF.

**34.** A method of reducing tumor cell growth in an animal comprising introducing a vector comprising an expression cassette of claim 1 into an animal, wherein the first or second transgene is polypeptide-encoding sequence encoding an anti-angiogenic polypeptide, and causing the anti-angiogenic polypeptide to be expressed by a cell of the animal.

**35.** A method of reducing tumor cell growth in an animal comprising introducing a vector comprising an expression cassette of claim 18 into an animal, wherein the first or second polypeptide-encoding sequence encodes a tumor suppressor polypeptide, and causing the tumor suppressor polypeptide to be expressed by a cell of the animal.

**36.** A method of producing a polypeptide comprising introducing a vector comprising an expression cassette of claim 1 into the cell, wherein the expression cassette contains the sequence encoding the polypeptide linked to appropriate regulatory regions, causing the polypeptide to be expressed by the cell, and isolating the polypeptide from the cell.

**37.** An expression cassette for expressing transgenes in mammalian cells or animals, comprising in order from 5' to 3', a transcriptional promoter, an intron sequence, a first polypeptide-encoding transgene sequence, a poly A site, a poly A site, a second polypeptide-encoding transgene sequence, an intron sequence, and a transcriptional promoter, wherein the expression levels in a mammalian cell result in an amount of polypeptide per cell of the first transgene within about 3 times the level of the second transgene.

**38.** The expression cassette of claim 37, wherein a promoter is the immediate early gene promoter of CMV.

**39.** The expression cassette of claim 37, wherein a promoter is the promoter of the human elongation factor-1α gene.

**40.** The expression cassette of claim 37, wherein a poly A site is the SV40 late poly A site.

**41.** The expression cassette of claim 37, wherein a poly A site is the bovine growth hormone poly A site.

**42.** A plasmid comprising an expression cassette of one of claims 37-41.

**43.** An adeno-associated virus vector comprising an expression cassette of one of claims 37-41.

**44.** An adenovirus vector comprising an expression cassette of one of claims 37-41.

**45.** A cell comprising a plasmid or vecor of one of claims 46-48, or progeny thereof.

**46.** An animal comprising a plasmid or vector of one of claims 42-44, or progeny thereof.

**47.** The expression cassette of claim 37, wherein the expression levels in a mammalian cell result in an amount of polypeptide per cell of the first transgene within about 100% of the level of the second transgene.

**48.** The expression cassette of claim 37, wherein the expression levels in a mammalian cell result in an amount of polypeptide per cell of the first transgene within about 75% of the level of the second transgene.

**49.** The expression cassette of claim 37, wherein the expression levels in a mammalian cell result in an amount of polypeptide per cell of the first transgene within about 50% of the level of the second transgene.

**50.** The expression cassette of claim 1, further comprising a third transgene.

**51.** The expression cassette of claim 50, wherein the transgenes are a TK encoding nucleic acid, an IL-2 encoding nucleic acid, and a GM-CSF encoding nucleic acid.

**52.** The expression cassette of claim 50, wherein the transgenes are selected from a suicide gene and a cytokine gene.

**53.** The expression cassette of claim 50 that is an adenovirus vector.

**54.** The expression cassette of claim 37, further comprising a third polypeptide-encoding transgene sequence.

**55.** The expression cassette of claim 54, wherein the polypeptides are selected from suicide proteins and cytokines.

**56.** The expression cassette of claim 54, wherein the polypeptides are IL-2, GM-CSF, and TK.

**57.** The expression cassette of claim 54 that is an adenovirus vector.

**58.** A method for inhibiting tumor cell metastasis in a mammal comprising introducing one or more vectors into a tumor cell or an area surrounding a tumor cell, wherein at least one of the vectors comprises the cassette of one of claims 1, 37, 50, or 54, and wherein the transgenes are selected from suicide genes and cytokines, and allowing the transgenes to be expressed in or surrounding the tumor cell.

**57.** The method of claim 58, wherein one or more vector is an adenovirus vector.

**58.** The method of claim 58, wherein the transgenes are IL-2 and GM-CSF.

**59.** The method of claim 58, wherein the transgenes are IL-2, GM-CSF, and TK.

**60.** The method of claim 59, wherein each of the one or more vectors is an adenoviral vector.

**61.** A method of improving or producing immunity to a tumor in a mammal, comprising introducing a vector comprising an expression cassette of one of claims 1 or 37, wherein one of the transgenes is a suicide gene or apoptotic gene and a second transgene is a cytokine, and allowing the tumor cells lysed or killed by the activity of the suicide or apoptotic protein expressed from the suicide gene or apoptotic gene to produce an immune response to the tumor.

**62.** The method of claim 61, wherein the suicide gene is a TK gene.

**63.** The method of claim 61, wherein the cytokine gene is GM-CSF.

**64.** The method of claim 61, wherein the cytokine gene is IL-2.

**65.** The method of claim 61, wherein two separate vectors are used.

**66.** The method of claim 65, wherein the vectors together encode a TK gene, an IL-2, and a GM-CSF.

**67.** The method of claim 65, wherein the vectors are adenoviral vectors.

**68.** The expression cassette of claim 37, further comprising a third transgene.

**69.** The expression cassette of claim 68, wherein the transgenes encode a TK, an IL-2, and a GM-CSF.

**70.** The expression cassette of claim 69 that is an adenovirus vector.

**71.** The expression cassette of claim 69 that is a plasmid vector.
